# EUROPEAN PATENT APPLICATION

(11) **EP 1 887 004 A1**
(43) Date of publication of application: **13.02.2008**
(21) Application number: 06380225.0
(22) Date of filing: 07.08.2006
(51) Int. Cl.: C07D 403/06, A61K 31/517

(54) **Crystalline forms of (1R,2R)-7-Chloro-3-[2-(2,4-difluorophenyl)-2-hydroxy-1-methyl-3-(1H-1,2,4-triazol-1-yl)propyl]quinazolin-4(3H)-one**

(71) Applicant: Palau Pharma, S.A., 08184 Palau-solità i Plegamans (ES)
(72) Inventor: Bartroli Orpi, Javier, 08034 Barcelona (ES); Molins Grau, Elies, 08193 Bellaterra (ES); Roig Serra, Anna, 08193 Bellaterra (ES); Canovas Paredes, Antonio, 08551 Tona (ES)
(74) Representative: Evens, Paul Jonathan

(57) **Abstract**

Novel crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one, pharmaceutical compositions containing these crystalline forms, methods of using these crystalline forms for treating and/or preventing various microbial and/or fungal infections or disorders, and processes for obtaining these crystalline forms. In particular, the present subject matter relates to the specific crystalline Forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one.

## Description

### FIELD OF THE INVENTION

The present subject matter relates to novel crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one, pharmaceutical compositions containing these crystalline forms, methods of using these crystalline forms for treating and/or preventing various microbial and/or fungal infections or disorders, and processes for obtaining these crystalline forms. In particular, the present subject matter relates to the specific crystalline Forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one.

### BACKGROUND OF THE INVENTION

U.S. Patent No. 5,807,854 discloses various novel antifungal compounds of the formula I: 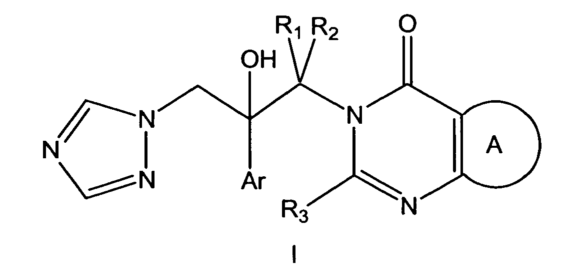 methods of making these compounds, pharmaceutical compositions containing these compounds, and their use in the treatment or prevention of fungal infections in animals. One of the specifically exemplified compounds falling within this genus is (1R,2R)-7-chloro-3-[2-(2,4-difluorophenyl)-2-hydroxy-1-methyl-3-(1H-1,2,4-triazol-1-yl)propyl]quinazolin-4(3H)-one. However, this patent does not disclose, refer to, or even suggest the benefits to obtaining specific crystalline forms of the compounds of formula I.

To prepare pharmaceutical compositions containing (1R,2R)-7-chloro-3-[2-(2,4-difluorophenyl)-2-hydroxy-1-methyl-3-(1H-1,2,4-triazol-1-yl)propyl]quinazolin-4(3H)-one for administration to mammals in accordance with exacting health registration requirements of the U.S. and international health registration authorities, e.g. the U.S. Food and Drug Administration's Good Manufacturing Practice ("GMP") requirements, there is a need to produce (1R,2R)-7-chloro-3-[2-(2,4-difluorophenyl)-2-hydroxy-1-methyl-3-(1H-1,2,4-triazol-1-yl)propyl]quinazolin-4(3H)-one in as pure a form as possible, especially a form having constant and consistent physical properties.

### SUMMARY OF THE INVENTION

Accordingly, the present subject matter provides unique solid forms of (1R,2R)-7-chloro-3-[2-(2,4-difluorophenyl)-2-hydroxy-1-methyl-3-(1H-1,2,4-triazol-1-yl)propyl]quinazolin-4(3H)-one represented by formula II:

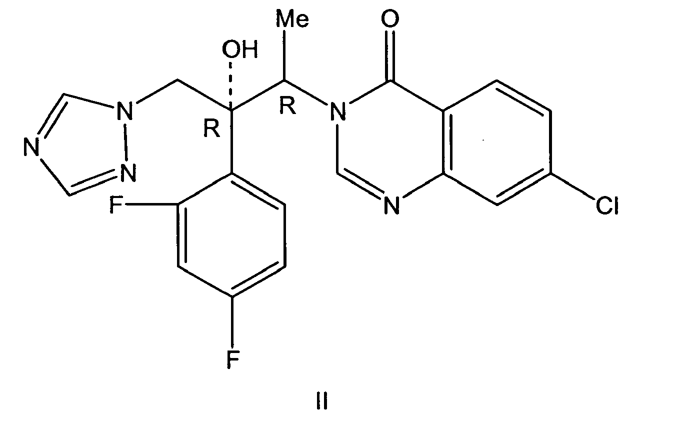

This compound is currently known to exist in either an amorphous form or as one of five crystalline forms that have presently been identified. Accordingly, a crystalline form of this compound is contemplated herein, such as a pure crystalline form substantially devoid of the compound's amorphous form and any residual solvent. In this regard, substantially pure crystalline forms of each of Forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one are contemplated herein.

In this regard, a preferred embodiment of the present subject matter relates to a crystalline form of (1R,2R)-7-chloro-3-[2-(2,4-difluorophenyl)-2-hydroxy-1-methyl-3-(1H-1,2,4-triazol-1-yl)propyl]quinazolin-4(3H)-one comprising the reaction product of (1R,2R)-7-chloro-3-[2-(2,4-difluorophenyl)-2-hydroxy-1-methyl-3-(1H-1,2,4-triazol-1-yl)propyl]quinazolin-4(3H)-one and an organic solvent.

Another preferred embodiment of the present subject matter relates to a substantially pure crystalline Form I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one.

Still another preferred embodiment of the present subject matter relates to a pharmaceutical composition comprising an anti-microbially or anti-fungally effective amount of a substantially pure crystalline Form I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one or a pharmaceutically acceptable salt or derivative thereof and a pharmaceutically acceptable carrier. The crystalline Form III or Form IV is particularly preferred in this regard.

Still yet another preferred embodiment of the present subject matter relates to a method of treating and/or preventing microbial and/or fungal infections in a mammal comprising administering to a mammal in need thereof an effective amount of a substantially pure crystalline Form I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one or a pharmaceutically acceptable salt or derivative thereof. The crystalline Form III or Form IV is particularly preferred in this regard.

Still yet another preferred embodiment of the present subject matter relates to a method of treating and/or preventing Chagas Disease in a mammal comprising administering to a mammal in need thereof an effective amount of a substantially pure crystalline Form I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one or a pharmaceutically acceptable salt or derivative thereof. The crystalline Form III or Form IV is particularly preferred in this regard.

In still another preferred embodiment, the present subject matter relates to a process for preparing a crystalline Form I or II of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
crystallizing a crystalline Form I or II of (1R,2R)-7-chloro-3-[2-(2,4-difluorophenyl)-2-hydroxy-1-methyl-3-(1H-1,2,4-triazol-1-yl)propyl]quinazolin-4(3H)-one from an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one using supercritical CO₂ crystallization conditions.

Yet another preferred embodiment of the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
adding an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one to a solvent selected from the group consisting of ethanol, ethyl acetate, dichloromethane, and a combination of ethanol and ethyl acetate to form a solution or suspension; and
crystallizing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one from said solution or suspension.

Moreover, another preferred embodiment of the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in a solvent selected from the group consisting of ethanol, ethyl acetate, dichloromethane, and a combination of ethanol and ethyl acetate to form a solution;
crystallizing crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one; and
drying said crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

In a particularly preferred embodiment in this regard, the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. However, any form of (1R,2R)-7-chloro-3-[2-(2,4-difluorophenyl)-2-hydroxy-1-methyl-3-(1H-1,2,4-triazol-1-yl)propyl]quinazolin-4(3H)-one may be suitable for these purposes.

Still another preferred embodiment of the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
adding (1R,2R)-7-chloro-3-[2-(2,4-difluorophenyl)-2-hydroxy-1-methyl-3-(1H-1,2,4-triazol-1-yl)propyl]quinazolin-4(3H)-one to an ethanol solvent to form a solution or suspension;
crystallizing crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one; and
drying said crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

In another preferred embodiment, the present subject matter relates to a process for preparing a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
storing an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one for 3 months at 40°C and 75% HR;
transforming some of said amorphous (1R,2R)-7-chloro-3-[2-(2,4-difluorophenyl)-2-hydroxy-1-methyl-3-(1H-1,2,4-triazol-1-yl)propyl]quinazolin-4(3H)-one to a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one during storage; and
obtaining said crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

In another preferred embodiment, the present subject matter relates to a process for preparing a crystalline Form IV of (1R,2R)-7-chloro-3-[2-(2,4-difluorophenyl)-2-hydroxy-1-methyl-3-(1H-1,2,4-tiazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in ethanol to form a solution;
adding the solution to water to form a suspension;
stirring the suspension for more than 30 minutes;
obtaining crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one; and
separating said crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

In a particularly preferred embodiment in this regard, the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. However, any form of (1R,2R)-7-chloro-3-[2-(2,4-difluorophenyl)-2-hydroxy-1-methyl-3-(1H-1,2,4-triazol-1-yl)propyl]quinazolin-4(3H)-one may be suitable for these purposes.

In another preferred embodiment, the present subject matter relates to a process for preparing a crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in ethyl acetate to form a solution;
adding hexane to the solution;
optionally adding diethyl ether to the solution;
crystallizing crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one; and
separating said crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

In a particularly preferred embodiment in this regard, the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. However, any form of (1R,2R)-7-chloro-3-[2-(2,4-difluorophenyl)-2-hydroxy-1-methyl-3-(1H-1,2,4-triazol-1-yl)propyl]quinazolin-4(3H)-one may be suitable for these purposes.

### BRIEF DESCRIPTION OF THE FIGURES

Fig. 1 presents a characteristic X-ray powder diffraction (XRPD) pattern of the crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 2 presents a characteristic infrared (IR) spectrum of the crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 3 presents a characteristic differential scanning calorimetry (DSC) thermogram of the crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 4 presents a characteristic XRPD pattern of the crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 5 presents a characteristic IR spectrum of the crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 6 presents a characteristic DSC thermogram of the crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 7 presents a characteristic XRPD pattern of the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 8 presents a characteristic IR spectrum of the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 9 presents a characteristic DSC thermogram of the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 10 presents a characteristic XRPD pattern of the crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 11 presents a characteristic IR spectrum of the crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 12 presents a characteristic DSC thermogram of the crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 13 presents a characteristic XRPD pattern of the crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 14 presents a characteristic IR spectrum of the crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

Fig. 15 presents a characteristic DSC thermogram of the crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

### DETAILED DESCRIPTION OF THE INVENTION

### Definitions

As used herein, the terms "administering", "administration", and like terms refer to any method which, in sound medical or cosmetic practice, delivers the composition to a subject in such a manner as to provide a net positive effect.

The phrase "crystalline form" as used herein refers to crystals of the same molecule having different physical properties as a result of the order of the molecules in the crystal lattice. Accordingly, as used herein the phrase "crystalline form" is intended to refer to different crystalline forms, polymorphs, pseudopolymorphs, and solvate forms of a single molecular entity. Different crystalline forms of a single compound may have different chemical, physical, mechanical, electrical, thermodynamic, and/or biological properties from each other. The differences in physical properties exhibited by crystalline forms affect pharmaceutical parameters such as storage stability, compressibility, density (important in formulation and product manufacturing), dissolution rates (an important factor in determining bio-availability), solubility, melting point, chemical stability, physical stability, powder flowability, compaction, and particle morphology. Each separate crystalline form of an individual compound will exhibit consistent chemical, physical, mechanical, electrical, thermodynamic, and biological properties.

Differences in stability can result from changes in chemical reactivity (e.g. differential oxidation, such that a dosage form discolors more rapidly when comprised of one crystalline form than when comprised of another crystalline form) or mechanical changes (e.g. tablets crumble on storage as a kinetically favored crystalline form converts to thermodynamically more stable crystalline form) or both (e.g. tablets of one crystalline form are more susceptible to breakdown at high humidity). As a result of solubility/dissolution differences, some crystalline form transitions affect potency and/or toxicity. In addition, the physical properties of the crystal may be important in processing; for example, one crystalline form might be more likely to form solvates or might be difficult to filter and wash free of impurities (i.e. particle shape and size distribution might be different between one crystalline form relative to the other).

As used herein, the phrases an "effective amount" or a "therapeutically effective amount" of an active agent or ingredient, or pharmaceutically active agent or ingredient, which are synonymous herein, refer to an amount of the pharmaceutically active agent sufficient enough to have a net positive effect upon administration. A therapeutically effective amount of the pharmaceutically active agent will cause a substantial relief of symptoms when administered repeatedly over time. Effective amounts of the pharmaceutically active agent will vary with the particular condition or conditions being treated, the severity of the condition, the duration of the treatment, the specific components of the composition being used, and like factors.

As used herein, the phrase "pharmaceutically acceptable salts" refers to salts of certain ingredient(s) which possess the same activity as the unmodified compound(s) and which are neither biologically nor otherwise undesirable. A salt can be formed with, for example, organic or inorganic acids. Non-limiting examples of suitable acids include acetic acid, acetylsalicylic acid, adipic acid, alginic acid, ascorbic acid, aspartic acid, benzoic acid, benzenesulfonic acid, bisulfic acid, boric acid, butyric acid, camphoric acid, camphorsulfonic acid, carbonic acid, citric acid, cyclopentanepropionic acid, digluconic acid, dodecylsulfic acid, ethanesulfonic acid, formic acid, fumaric acid, glyceric acid, glycerophosphoric acid, glycine, glucoheptanoic acid, gluconic acid, glutamic acid, glutaric acid, glycolic acid, hemisulfic acid, heptanoic acid, hexanoic acid, hippuric acid, hydrobromic acid, hydrochloric acid, hydroiodic acid, hydroxyethanesulfonic acid, lactic acid, maleic acid, malic acid, malonic acid, mandelic acid, methanesulfonic acid, mucic acid, naphthylanesulfonic acid, naphthylic acid, nicotinic acid, nitrous acid, oxalic acid, pelargonic, phosphoric acid, propionic acid, saccharin, salicylic acid, sorbic acid, succinic acid, sulfuric acid, tartaric acid, thiocyanic acid, thioglycolic acid, thiosulfuric acid, tosylic acid, undecylenic acid, and naturally and synthetically derived amino acids.

If organic bases are used, poorly volatile bases are preferably employed, for example low molecular weight alkanolamines such as ethanolamine, diethanolamine, N-ethylethanolamine, N-methyldiethanolamine, triethanolamine, diethylaminoethanol, 2-amino-2-methyl-n-propanol, dimethylaminopropanol, 2-amino-2-methylpropanediol, and triisopropanolamine. Ethanolamine is particularly preferred in this regard. Further poorly volatile bases which may be mentioned are, for example, ethylenediamine, hexamethylenediamine, morpholine, piperidine, piperazine, cyclohexylamine, tributylamine, dodecylamine, N,N-dimethyldodecylamine, stearylamine, oleylamine, benzylamine, dibenzylamine, N-ethylbenzylamine, dimethylstearylamine, N-methylmorpholine, N-methylpiperazine, 4-methylcyclohexylamine, and N-hydroxyethylmorpholine.

Salts of quaternary ammonium hydroxides such as trimethylbenzylammonium hydroxide, tetramethylammonium hydroxide, or tetraethylammonium hydroxide can also by used, as can guanidine and its derivatives, in particular its alkylation products. However, it is also possible to employ as salt-forming agents, for example, low molecular weight alkylamines such as methylamine, ethylamine, or triethylamine. Suitable salts for the components to be employed according to the present subject matter are also those with inorganic cations, for example alkali metal salts, in particular sodium, potassium, or ammonium salts, alkaline earth metal salts such as, in particular, the magnesium or calcium salts, as well as salts with bi- or tetravalent cations, for example the zinc, aluminum, or zirconium salts. Also contemplated are salts with organic bases, such as dicyclohexylamine salts; methyl-D-glucamine; and salts with amino acids, such as arginine, lysine, and so forth. Also, the basic nitrogen-containing groups can be quaternized with such agents as lower alkyl halides, such as methyl, ethyl, propyl, and butyl chlorides, bromides, and iodides; dialkyl sulfates, such as dimethyl, diethyl, dibutyl, and diamyl sulfates; long chain halides, such as decyl, lauryl, myristyl, and stearyl chlorides, bromides, and iodides; asthma halides, such as benzyl and phenethyl bromides; and others. Water or oil-soluble or dispersible products are thereby obtained.

The phrase "reaction product" as used herein refers to any crystalline form obtained from the processes described herein, including but not limited to anhydrates, hydrates, polymorphs, solvates, N-oxides, and/or salts of these crystalline forms.

The phrase "substantially pure" as used herein refers to an individual crystalline form which is substantially devoid of all other crystalline forms, as well as degradation products of the crystalline form, the amorphous form, and any residual solvent, on a % weight basis, unless otherwise specified.

The term "treating" as used herein refers to the process of producing an effect on biological activity, function, health, or condition of an organism in which such activity is maintained, enhanced, diminished, or applied in a manner consistent with the general health and well-being of the organism.

Other terms as used herein are meant to be defined by their well-known meanings in the art.

### Crystalline Forms of (1R,2R)-7-chloro-3-[2-(2,4-difluorophenyl)-2-hydroxy-1-methyl-3-(1H-1,2,4-triazol-1-yl)propyl]quinazolin-4(3H)-one

Five different crystalline forms of the compound (1R,2R)-7-chloro-3-[2-(2,4-difluorophenyl)-2-hydroxy-1-methyl-3-(1H-1,2,4-triazol-1-yl)propyl]quinazolin-4(3H)-one have presently been identified: crystalline Forms I, II, III, IV, and V. Of these different crystalline forms, the most preferred will provide an antifungal, antimicrobial drug having the lowest impurity content, most consistent product quality, most consistent physical characteristics including color, rate of dissolution, and ease of handling, and greatest long term stability in comparison to the other crystalline forms or the amorphous form.

Accordingly, the present subject matter relates to methods of identifying, obtaining, and purifying the various crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one. These crystalline forms, namely Forms I-V, were identified as five distinct crystal forms. Certain physical characteristics of these crystalline forms generated during crystallization studies, are as follows:
• Form I: typically obtained by crystallizing the amorphous form using supercritical CO₂ crystallization conditions. This form displays a characteristic XRPD pattern, a characteristic IR spectrum, and a characteristic DSC profile.
• Form II: typically obtained by crystallizing the amorphous form using supercritical CO₂ crystallization conditions. This form displays a characteristic XRPD pattern, a characteristic IR spectrum, and a characteristic DSC profile.
• Form III: obtained under standard crystallization conditions using a variety of solvents such as ethanol, ethyl acetate, dichloromethane, and a combination of ethanol and ethyl acetate. This form displays a characteristic XRPD pattern, a characteristic IR spectrum, and a DSC profile with a strong endothermic peak onset at about 99°C. No degradation products of form III were detected after 6 months of storage at 30°C/65% RH and 25°C/60% RH.
• Form IV: typically obtained by first dissolving the amorphous form in ethanol, then suspending this solution in water and stirring for a certain amount of time. This form may also be obtained directly from the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one after storage for an extended period of time, for example after storage for 3 months at 40°C and 75% HR, as some but not all of the amorphous form converts to form IV. This form displays a characteristic XRPD pattern, a characteristic IR spectrum, and a DSC profile with a strong endothermic peak onset at about 121°C.
• Form V: obtained under standard crystallization conditions, typically by first dissolving the amorphous form in ethyl acetate, then adding hexane to this solution. Diethyl ether can then be optionally added. This form displays a characteristic XRPD pattern, a characteristic IR spectrum, and a DSC profile with a strong endothermic peak onset at about 108°C. Form V can best be characterized as a crystalline phase containing between about 2 and about 7% by weight of ethyl acetate and about 0.5 to about 2.5% by weight of hexane.

### Purity

The present subject matter contemplates substantially pure crystalline Forms I, II, III, IV, and/or V of (1R,2R)-7-chloro-3-[2-(2,4-difluorophenyl)-2-hydroxy-1-methyl-3-(1H-1,2,4-triazol-1-yl)propyl]quinazolin-4(3H)-one. In this regard, the present subject matter contemplates each of these crystalline forms which is substantially devoid of the amorphous form and of any residual solvent on a weight % basis. In a preferred embodiment, the present subject matter particularly contemplates each of these crystalline forms free of any residual solvent. In an alternative preferred embodiment, the present subject matter contemplates each of these crystalline forms further substantially devoid of the other crystalline forms.

For example, the present subject matter contemplates crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one which has less than about 10% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one which has less than about 10% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one which has less than about 10% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one which has less than about 10% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; and crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one which has less than about 10% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis. Accordingly, the present subject matter contemplates each of these crystalline forms having at least 90% purity as defined by X-ray powder diffraction.

In an alternative example, the present subject matter further contemplates crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one which also has less than about 10% by weight of the crystalline forms II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one which also has less than about 10% by weight of the crystalline forms I, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one which also has less than about 10% by weight of the crystalline forms I, II, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one which also has less than about 10% by weight of the crystalline forms I, II, III, or V of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis; and crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one which also has less than about 10% by weight of the crystalline forms I, II, III, or IV of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis.

In a preferred embodiment, the present subject matter contemplates each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one which have less than about 7% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis, or have a 93% purity as defined by X-ray powder diffraction. In an alternative preferred embodiment in this regard, the present subject matter may further contemplate each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one which also have less than about 7% by weight of the other crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis.

In a particularly preferred embodiment, the present subject matter contemplates each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one which have less than about 5% by weight of any residual solvent and the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis, or have a 95% purity as defined by X-ray powder diffraction. In an alternative preferred embodiment in this regard, the present subject matter may further contemplate each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one which also have less than about 5% by weight of the other crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a weight % basis.

In another preferred embodiment, the present subject matter contemplates each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one which have less than about 10% by weight of any residual solvent. In a further preferred embodiment in this regard, the present subject matter contemplates each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one which have less than 7%, more preferably less than 5%, or even more preferably less than 2%, by weight of any residual solvent. In a most preferred embodiment, the crystalline form free of residual solvent is crystalline Form III.

Additionally, the present subject matter contemplates substantially pure crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one substantially devoid of degradation products of the (1R,2R)-7-chloro-3-[2-(2,4-difluorophenyl)-2-hydroxy-1-methyl-3-(1H-1,2,4-triazol-1-yl)propyl]quinazolin-4(3H)-one. In this regard, the present subject matter further contemplates a substantially pure crystalline Form I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one having not less than 97% purity with respect to other forms of the compound, any degradation products, or with respect to any residual solvent.

### Physical Characteristics

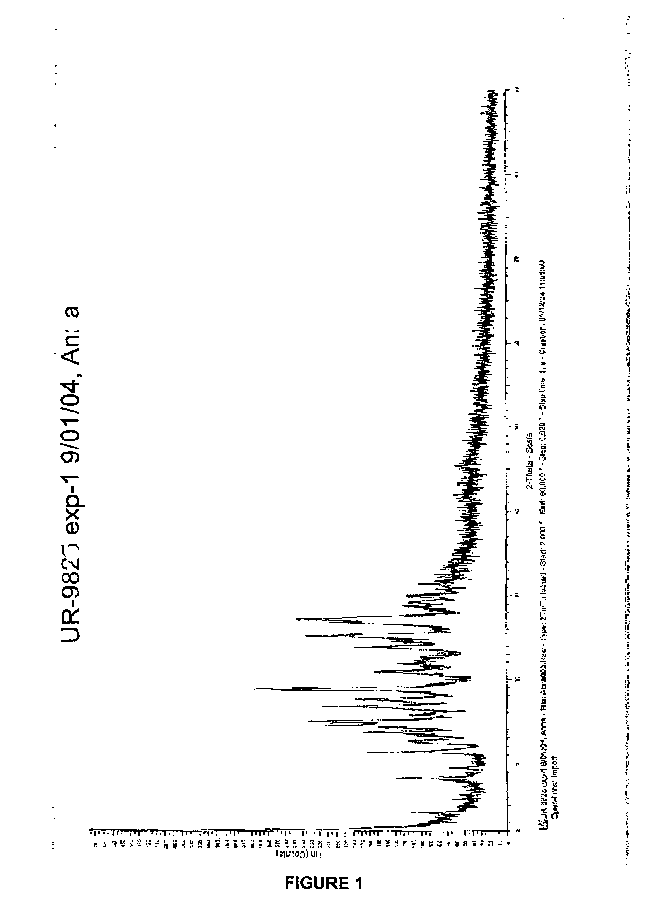

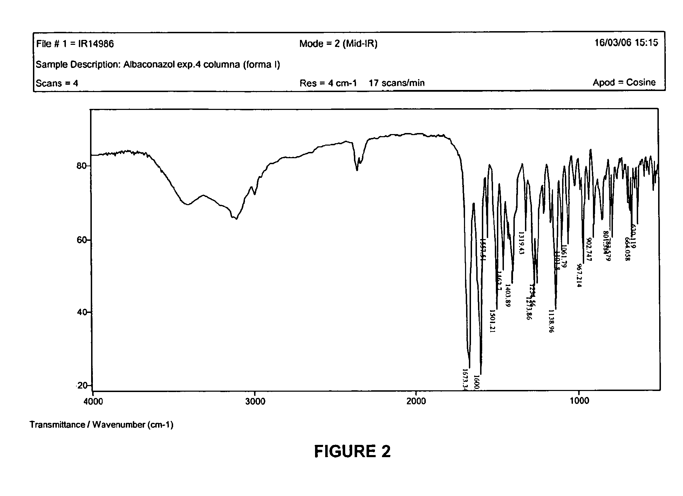

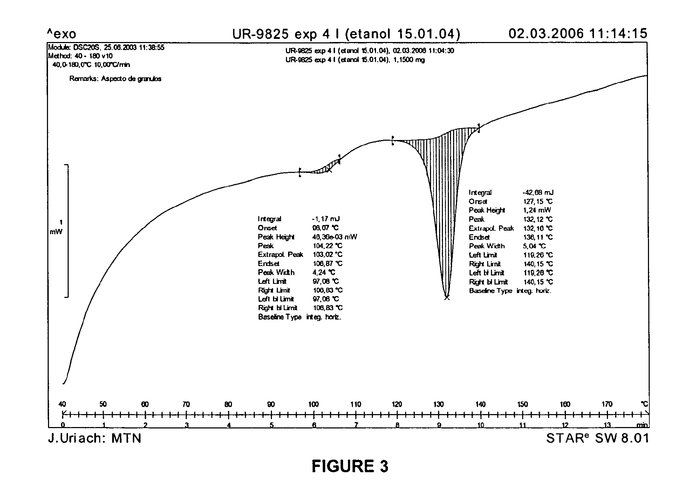

The five crystalline forms of (1R,2R)-7-chloro-3-[2-(2,4-difluorophenyl)-2-hydroxy-1-methyl-3-(1H-1,2,4-triazol-1-yl)propyl]quinazolin-4(3H)-one designated as Form I, II, III, IV, and V can readily be distinguished from one another by examination of their respective characteristic x-ray diffraction (XRPD) patterns (see Figs 1, 4, 7, 10, and 13, respectively), characteristic IR spectra (see Figs. 2, 5, 8, 11, and 14, respectively), or differential scanning calorimetry (DSC) thermograms (see Fig. 3, 6, 9, 12, and 15, respectively).

### Form I

Crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one was in part identified by its characteristic XRPD pattern. The XRPD pattern for Form I was measured on a D5000 Siemens X-ray diffractometer, having a graphite secondary monochromator and a scintillation detector. The anode was of copper (wavelength CuKa: 1.541838 A, V= 50 kV, I=20 mA) and the ambient temperature was kept at 21°C.

A characteristic XRPD pattern distinctive to Form I was observed, as represented by Fig. 1. The characteristic 2-theta positions and corresponding intensities observed for this specific pattern are summarized below in Table 1.

**TABLE 1**

| **Characteristic XRPD 2-Theta Positions and Intensities for Form I** | |
|---|---|
| **Characteristic XRPD 2-theta positions (+/-0.2)** | **Characteristic XRPD Intensities (counts)** |
| 4.11 | 115 |
| 8.22 | 128 |
| 9.39 | 66 |
| 11.29 | 161 |
| 12.41 | 102 |
| 13.21 | 95 |
| 13.62 | 160 |
| 14.43 | 206 |
| 14.93 | 231 |
| 15.7 | 104 |
| 16.74 | 215 |
| 17.3 | 134 |
| 17.57 | 178 |
| 18.79 | 296 |
| 20.88 | 151 |
| 21.88 | 125 |
| 22.62 | 98 |
| 23.64 | 119 |
| 23.82 | 177 |
| 25.2 | 234 |
| 26.77 | 206 |
| 27.21 | 245 |
| 28.57 | 116 |
| 29.16 | 113 |
| 29.97 | 110 |
| 30.75 | 81 |
| 31.35 | 93 |
| 45.28 | 56 |
| 48.87 | 42 |
| 55.02 | 36 |

The most relevant 2-theta positions and corresponding intensities observed for this specific XRPD pattern for Form I are summarized below in Table 2.

**TABLE 2**

| **Most Relevant XRPD 2-Theta Positions and Intensities for Form I** | |
|---|---|
| **Characteristic XRPD 2-theta positions (+/-0.2)** | **Characteristic XRPD Intensities (counts)** |
| 4.11 | 115 |
| 8.22 | 128 |
| 9.39 | 66 |
| 11.29 | 161 |
| 12.41 | 102 |
| 13.62 | 160 |
| 16.74 | 215 |
| 17.57 | 178 |
| 18.79 | 296 |
| 23.82 | 177 |
| 25.2 | 234 |

The complete infrared (IR) spectrum of the crystalline Form I is shown in Fig. 2 and is characterized as summarized below in Table 3. All IR spectrum analyses obtained and discussed herein were generated on a Bomem BM-100 spectrophotometer.

**TABLE 3**

| **Infrared Spectrum for Form I** |
|---|
| **Frequency (cm⁻¹) (+/- 0.1)** |
| 1673.3 |
| 1600.0 |
| 1557.5 |
| 1501.2 |
| 1462.7 |
| 1403.9 |
| 1319.4 |
| 1273.9 |
| 1254.6 |
| 1139.0 |
| 1101.8 |
| 1061.8 |
| 967.2 |
| 902.7 |
| 801.3 |
| 783.6 |
| 664.1 |
| 630.1 |

Form I was further observed to have a characteristic endothermic peak using DSC analysis, as represented by Fig. 3. All DSC analyses obtained herein were measured on a Mettler-Toledo DSC-20 instrument. The samples (each of about 1 mg), were enclosed in aluminium pans having small holes accurately weighted, heated over a temperature range of 40-180°C, at a scan rate of 10°/min, under a nitrogen purge. The apparatus was calibrated with (99.9% pure) indium.

### Form II

Crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one was likewise in part identified by its characteristic XRPD pattern. The XRPD pattern for Form II was measured on a D5000 Siemens X-ray diffractometer, having a graphite secondary monochromator and a scintillation detector. The anode was of copper (wavelength CuKa: 1.541838 A, V= 50 kV, I=20 mA) and the ambient temperature was kept at 21°C.

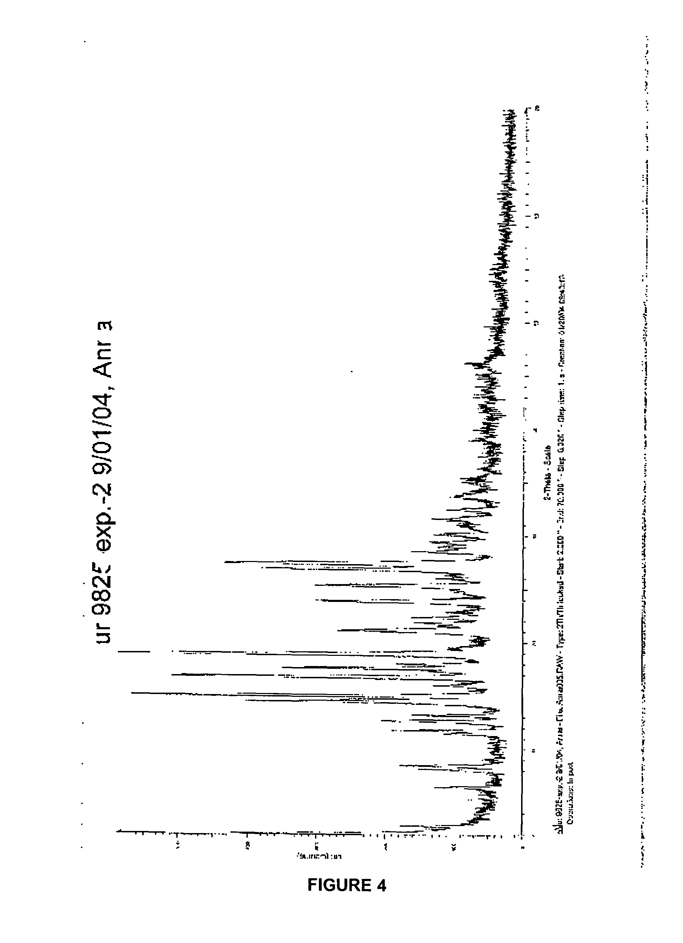

A characteristic XRPD pattern distinctive to Form II was observed, as represented by Fig. 4. The characteristic 2-theta positions and corresponding intensities observed for this specific pattern are summarized below in Table 4.

**TABLE 4**

| **Characteristic XRPD 2-Theta Positions and Intensities for Form II** | |
|---|---|
| **Characteristic XRPD 2-theta positions (+/-0.2)** | **Characteristic XRPD Intensities (counts)** |
| 2.72 | 176 |
| 5.31 | 62 |
| 6.35 | 126 |
| 7.98 | 124 |
| 8.37 | 177 |
| 9.01 | 56 |
| 11.4 | 112 |
| 11.7 | 188 |
| 12.6 | 208 |
| 13.15 | 160 |
| 14.42 | 402 |
| 14.98 | 568 |
| 15.93 | 128 |
| 16.77 | 511 |
| 17.56 | 348 |
| 17.91 | 181 |
| 18.86 | 589 |
| 19.37 | 108 |
| 21.08 | 272 |
| 21.78 | 147 |
| 22.31 | 161 |
| 22.82 | 115 |
| 23.84 | 300 |
| 25.32 | 294 |
| 26 | 125 |
| 26.83 | 379 |
| 27.35 | 432 |
| 28.5 | 161 |
| 28.96 | 122 |
| 29.38 | 147 |
| 30.14 | 120 |
| 31.58 | 131 |
| 32.41 | 109 |
| 33.63 | 105 |
| 34.94 | 77 |
| 46.1 | 85 |

The most relevant 2-theta positions and corresponding intensities observed for this specific XRPD pattern for Form II are summarized below in Table 5.

**TABLE 5**

| **Most Relevant XRPD 2-Theta Positions and Intensities for Form II** | |
|---|---|
| **Characteristic XRPD 2-theta positions (+/-0.2)** | **Characteristic XRPD Intensities (counts)** |
| 6.35 | 126 |
| 7.98 | 124 |
| 8.37 | 177 |
| 11.4 | 112 |
| 11.7 | 188 |
| 12.6 | 208 |
| 13.15 | 160 |
| 14.42 | 402 |
| 14.98 | 568 |
| 16.77 | 511 |
| 17.56 | 348 |
| 18.86 | 589 |
| 21.08 | 272 |
| 23.84 | 300 |
| 25.32 | 294 |
| 26.83 | 379 |
| 27.35 | 432 |

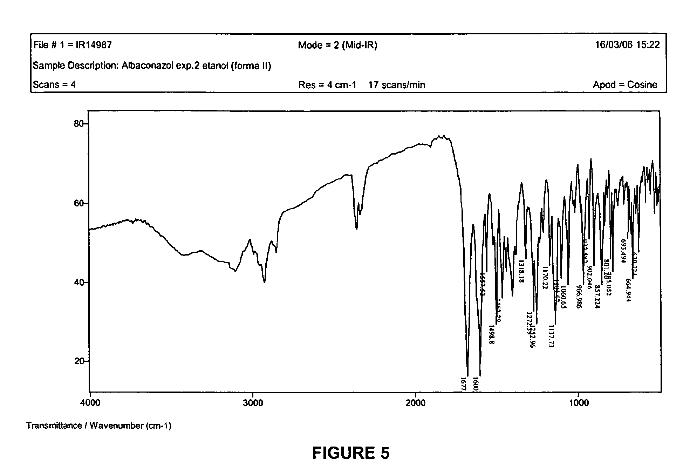

The complete infrared spectrum of the crystalline Form II is shown in Fig. 5 and is characterized as summarized below in Table 6.

**TABLE 6**

| **Infrared Spectrum for Form II** |
|---|
| **Frequency (cm⁻¹) (+/- 0.1)** |
| 1677.0 |
| 1600.0 |
| 1557.5 |
| 1498.8 |
| 1462.3 |
| 1318.2 |
| 1272.4 |
| 1253.0 |
| 1170.2 |
| 1137.7 |
| 1102.0 |
| 1060.7 |
| 967.0 |
| 932.6 |
| 902.0 |
| 857.2 |
| 801.3 |
| 785.1 |
| 693.5 |
| 664.9 |
| 630.7 |

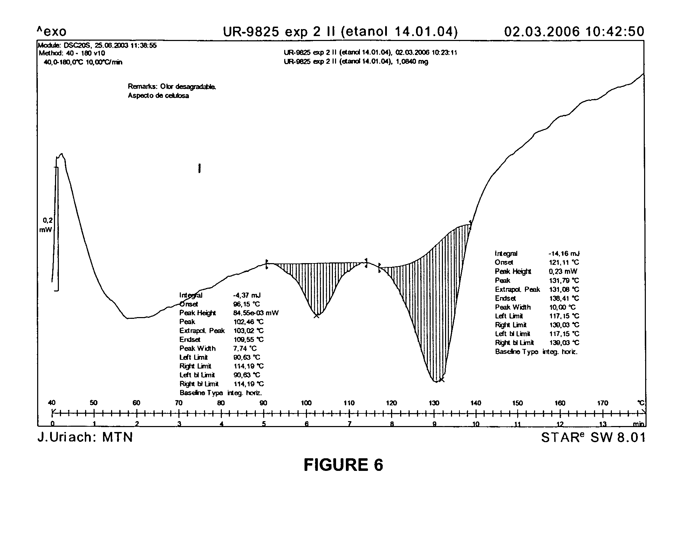

Form II was further observed to have a characteristic endothermic peak using DSC analysis, as represented by Fig. 6.

### Form III

Crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one was in part identified by its characteristic XRPD pattern. The XRPD pattern for Form III was measured at room temperature using a Philips X'Pert diffractometer equipped with a ?/2? goniometer, a Cu tube working at 50 kV and 40 mA (CuKa radiation, ? = 1.5419 A), a divergence slit = 1/4°, Soller slits = 0.04 rad, an anti-scatter slit = 1/4°, a receiving slit = 0.10 mm, and a secondary curved graphite monochromator. Data were collected in the range 2-35° of 2theta using a step-scan technique with a step size = 0.02° and a time per step = 20 s.

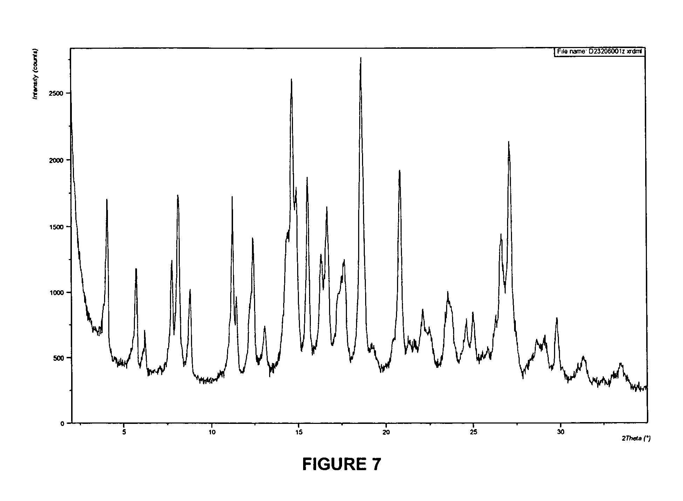

A characteristic XRPD pattern distinctive to Form III was observed, as represented by Fig. 7. The characteristic 2-theta positions and corresponding intensities observed for this specific pattern are summarized below in Table 7.

**TABLE 7**

| **Characteristic XRPD 2-Theta Positions and Intensities for Form III** | |
|---|---|
| **Characteristic XRPD 2-theta positions (+/-0.2)** | **Characteristic XRPD Relative Intensity (%)** |
| 4.08 | 51 |
| 5.73 | 34 |
| 6.22 | 13 |
| 7.77 | 36 |
| 8.15 | 60 |
| 8.80 | 30 |
| 11.25 | 61 |
| 11.47 | 29 |
| 12.44 | 48 |
| 13.09 | 17 |
| 14.33 | 46 |
| 14.68 | 98 |
| 14.89 | 60 |
| 15.57 | 68 |
| 16.35 | 43 |
| 16.68 | 58 |
| 17.27 | 29 |
| 17.63 | 40 |
| 18.66 | 100 |
| 19.32 | 12 |
| 20.85 | 71 |
| 22.12 | 25 |
| 22.49 | 18 |
| 23.58 | 29 |
| 24.63 | 20 |
| 25.02 | 24 |
| 26.65 | 48 |
| 27.12 | 77 |
| 28.74 | 15 |
| 29.11 | 17 |
| 29.81 | 23 |
| 31.35 | 10 |
| 33.48 | 8 |

The most relevant 2-theta positions and corresponding intensities observed for this specific XRPD pattern for Form III are summarized below in Table 8.

**TABLE 8**

| **Most Relevant XRPD 2-Theta Positions and Intensities for Form III** | |
|---|---|
| **Characteristic XRPD 2-theta positions (+/-0.2)** | **Characteristic XRPD Relative Intensity (%)** |
| 4.08 | 51 |
| 5.73 | 34 |
| 6.22 | 13 |
| 7.77 | 36 |
| 8.15 | 60 |
| 8.80 | 30 |
| 11.25 | 61 |
| 11.47 | 29 |
| 12.44 | 48 |
| 13.09 | 17 |
| 15.57 | 68 |
| 17.63 | 40 |
| 18.66 | 100 |
| 20.85 | 71 |
| 26.65 | 48 |
| 27.12 | 77 |

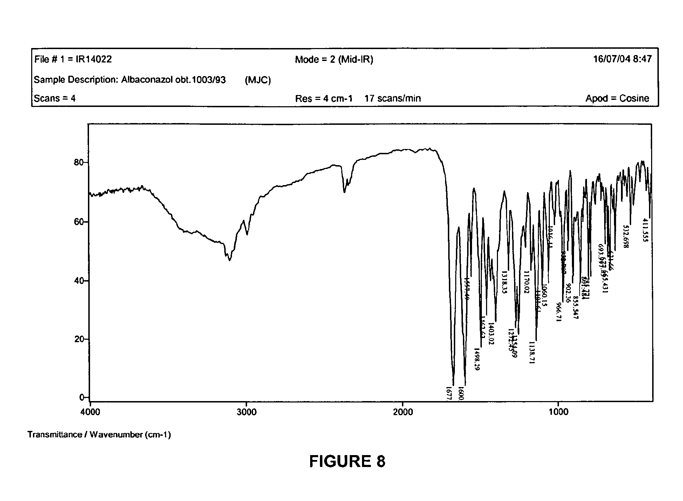

The complete infrared spectrum of the crystalline Form III is shown in Fig. 8 and is characterized as summarized below in Table 9.

**TABLE 9**

| **Infrared Spectrum for Form III** |
|---|
| **Frequency (cm⁻¹) (+/-0.1)** |
| 1677.0 |
| 1600.0 |
| 1557.5 |
| 1498.3 |
| 1462.6 |
| 1403.0 |
| 1318.4 |
| 1272.5 |
| 1254.1 |
| 1170.0 |
| 1138.7 |
| 1101.6 |
| 1060.2 |
| 1016.4 |
| 966.7 |
| 932.7 |
| 902.4 |
| 855.5 |
| 801.5 |
| 785.8 |
| 694.0 |
| 677.9 |
| 665.4 |
| 631.7 |
| 532.7 |
| 411.6 |

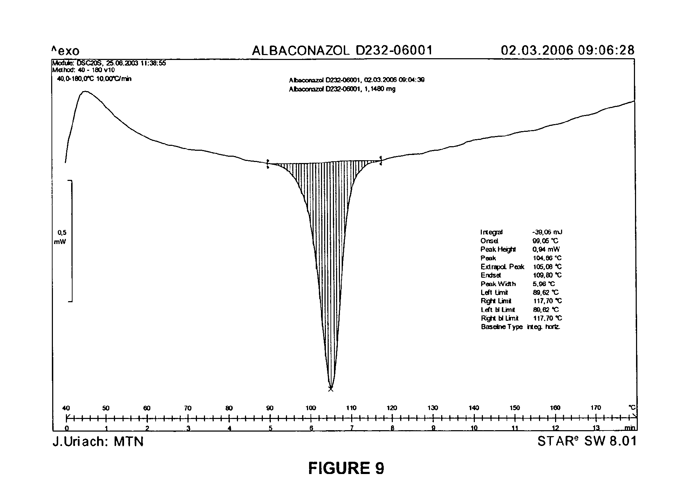

Form III was further observed to have a characteristic endothermic peak onset observed at about 99 +/- 5 degrees C using DSC analysis, as represented by Fig. 9.

### Form IV

Crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one was likewise in part identified by its characteristic XRPD pattern. The XRPD pattern for Form IV was measured at room temperature using a Philips X'Pert diffractometer equipped with a ?/2? goniometer, a Cu tube working at 50 kV and 40 mA (CuKa radiation, ? = 1.5419 A), a divergence slit = 1/4°, Soller slits = 0.04 rad, an anti-scatter slit = 1/4°, a receiving slit = 0.10 mm, and a secondary curved graphite monochromator. Data were collected in the range 2-35° of 2theta using a step-scan technique with a step size = 0.02° and a time per step = 20 s.

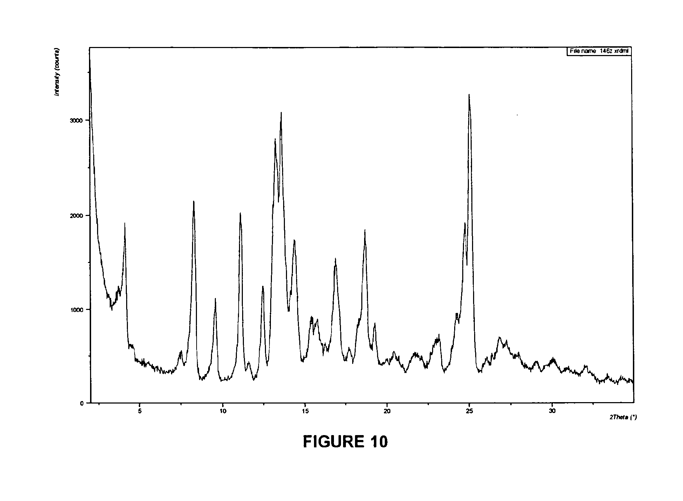

A characteristic XRPD pattern distinctive to Form IV was observed, as represented by Fig. 10. The characteristic 2-theta positions and corresponding intensities observed for this specific pattern are summarized below in Table 10.

**TABLE 10**

| **Characteristic XRPD 2-Theta Positions and Intensities for Form IV** | |
|---|---|
| **Characteristic XRPD 2-theta positions (+/-0.2)** | **Characteristic XRPD Relative Intensity (%)** |
| 3.74 | 14 |
| 4.15 | 39 |
| 7.5 | 8 |
| 8.33 | 64 |
| 9.61 | 30 |
| 11.16 | 60 |
| 11.61 | 7 |
| 12.49 | 34 |
| 13.29 | 84 |
| 13.64 | 96 |
| 14.41 | 51 |
| 15.43 | 22 |
| 15.74 | 22 |
| 16.90 | 42 |
| 17.71 | 12 |
| 18.25 | 20 |
| 18.74 | 51 |
| 19.30 | 21 |
| 20.43 | 11 |
| 21.78 | 10 |
| 23.20 | 15 |
| 24.26 | 24 |
| 24.78 | 57 |
| 25.11 | 100 |
| 26.03 | 8 |
| 26.86 | 16 |
| 27.25 | 15 |
| 28.00 | 10 |
| 29.05 | 7 |
| 30.07 | 8 |
| 30.91 | 5 |
| 32.05 | 5 |

The most relevant 2-theta positions and corresponding intensities observed for this specific XRPD pattern for Form IV are summarized below in Table 11.

**TABLE 11**

| **Most Relevant XRPD 2-Theta Positions and Intensities for Form IV** | |
|---|---|
| **Characteristic XRPD 2-theta positions (+/-0.2)** | **Characteristic XRPD Relative Intensity (%)** |
| 4.15 | 39 |
| 7.5 | 8 |
| 8.33 | 64 |
| 9.61 | 30 |
| 11.16 | 60 |
| 12.49 | 34 |
| 13.29 | 84 |
| 13.64 | 96 |
| 14.41 | 51 |
| 16.90 | 42 |
| 18.74 | 51 |
| 24.78 | 57 |
| 25.11 | 100 |

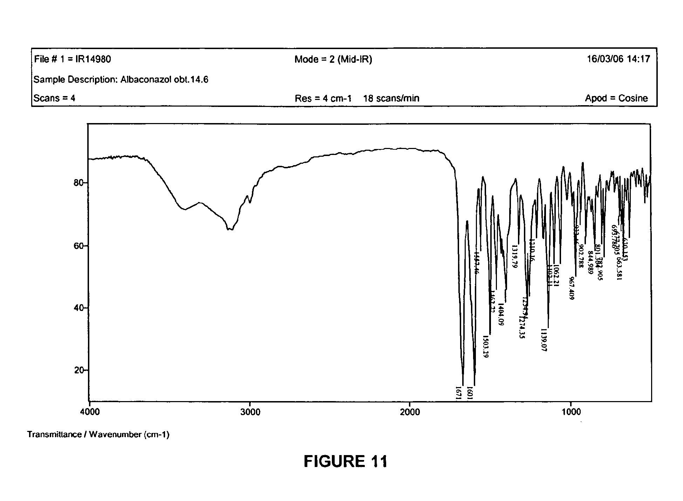

The complete infrared spectrum of the crystalline Form IV is shown in Fig. 11 and is characterized as summarized below in Table 12.

**TABLE 12**

| **Infrared Spectrum for Form IV** |
|---|
| **Frequency (cm⁻¹) (+/-0.1)** |
| 1671.0 |
| 1601.0 |
| 1557.5 |
| 1503.3 |
| 1462.7 |
| 1404.1 |
| 1319.8 |
| 1274.4 |
| 1254.9 |
| 1210.2 |
| 1139.1 |
| 1102.1 |
| 1062.2 |
| 967.4 |
| 933.5 |
| 902.8 |
| 845.0 |
| 801.4 |
| 782.9 |
| 693.8 |
| 677.7 |
| 663.6 |
| 630.2 |

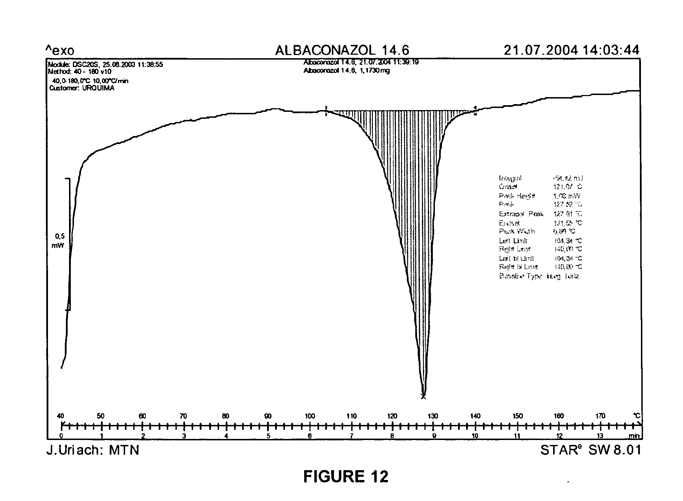

Form IV was further observed to have a characteristic endothermic peak onset observed at about 121 +/- 5 degrees C using DSC analysis, as represented by Fig. 12.

### Form V

Crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one was likewise in part identified by its characteristic XRPD pattern. The XRPD pattern for Form V was measured at room temperature using a Philips X'Pert diffractometer equipped with a ?/2? goniometer, a Cu tube working at 50 kV and 40 mA (CuKa radiation, ? = 1.5419 A), a divergence slit = 1/4°, Soller slits = 0.04 rad, an anti-scatter slit = 1/4°, a receiving slit = 0.10 mm, and a secondary curved graphite monochromator. Data were collected in the range 2-35° of 2theta using a step-scan technique with a step size = 0.02° and a time per step = 20 s.

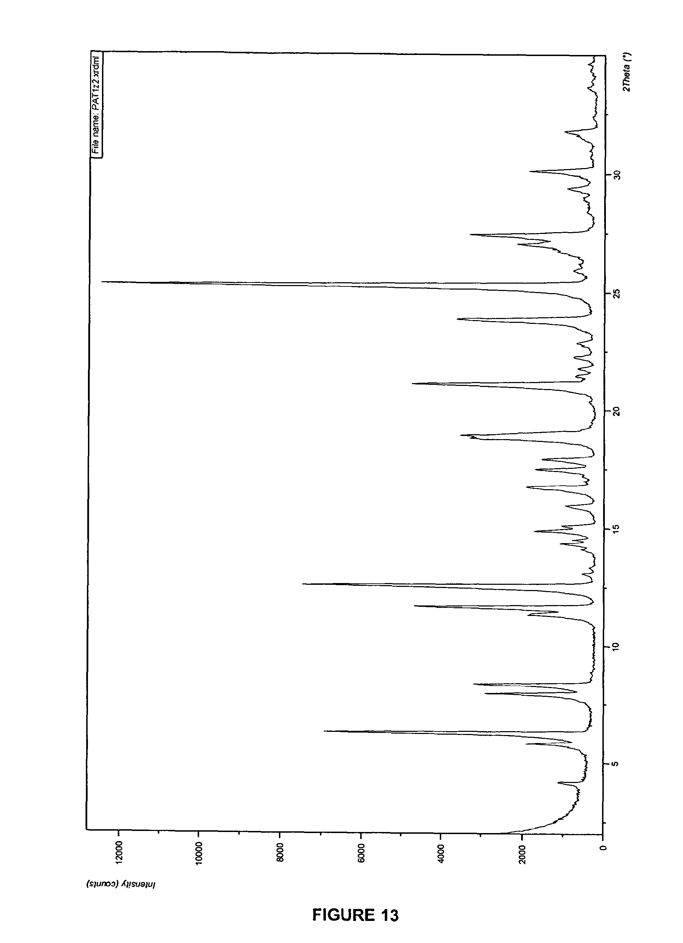

A characteristic XRPD pattern distinctive to Form V was observed, as represented by Fig. 13. The characteristic 2-theta positions and corresponding intensities observed for this specific pattern are summarized below in Table 13.

**TABLE 13**

| **Characteristic XRPD 2-Theta Positions and Intensities for Form V** | |
|---|---|
| **Characteristic XRPD 2-theta positions (+/-0.2)** | **Characteristic XRPD Relative Intensity (%)** |
| 4.17 | 5 |
| 5.83 | 12 |
| 6.28 | 51 |
| 7.96 | 21 |
| 8.35 | 23 |
| 11.35 | 13 |
| 11.66 | 36 |
| 12.57 | 59 |
| 13.06 | 2 |
| 13.34 | 1 |
| 14.11 | 3 |
| 14.34 | 7 |
| 14.52 | 4 |
| 14.89 | 12 |
| 15.09 | 7 |
| 15.95 | 5 |
| 16.74 | 14 |
| 16.98 | 2 |
| 17.21 | 3 |
| 17.49 | 12 |
| 17.91 | 11 |
| 18.82 | 25 |
| 18.9 | 27 |
| 20.36 | 1 |
| 21.1 | 37 |
| 21.47 | 4 |
| 21.79 | 4 |
| 22.27 | 4 |
| 22.88 | 4 |
| 23.86 | 28 |
| 25.3 | 100 |
| 25.95 | 5 |
| 26.2 | 3 |
| 26.74 | 7 |
| 27.04 | 16 |
| 27.44 | 25 |
| 28.43 | 2 |
| 28.98 | 2 |
| 29.41 | 6 |
| 30.13 | 13 |
| 30.73 | 1 |
| 30.98 | 1 |
| 31.78 | 7 |
| 32.36 | 1 |
| 33.61 | 2 |
| 33.9 | 1 |
| 34.35 | 1 |
| 34.62 | 2 |

The most relevant 2-theta positions and corresponding intensities observed for this specific XRPD pattern for Form V are summarized below in Table 14.

**TABLE 14**

| **Most Relevant XRPD 2-Theta Positions and Intensities for Form V** | |
|---|---|
| **Characteristic XRPD 2-theta positions (+/-0.2)** | **Characteristic XRPD Relative Intensity (%)** |
| 4.17 | 5 |
| 5.83 | 12 |
| 6.28 | 51 |
| 7.96 | 21 |
| 8.35 | 23 |
| 11.35 | 13 |
| 11.66 | 36 |
| 12.57 | 59 |
| 14.34 | 7 |
| 14.89 | 12 |
| 15.95 | 5 |
| 16.74 | 14 |
| 17.49 | 12 |
| 18.9 | 27 |
| 21.1 | 37 |
| 23.86 | 28 |
| 25.3 | 100 |
| 27.04 | 16 |
| 27.44 | 25 |
| 30.13 | 13 |

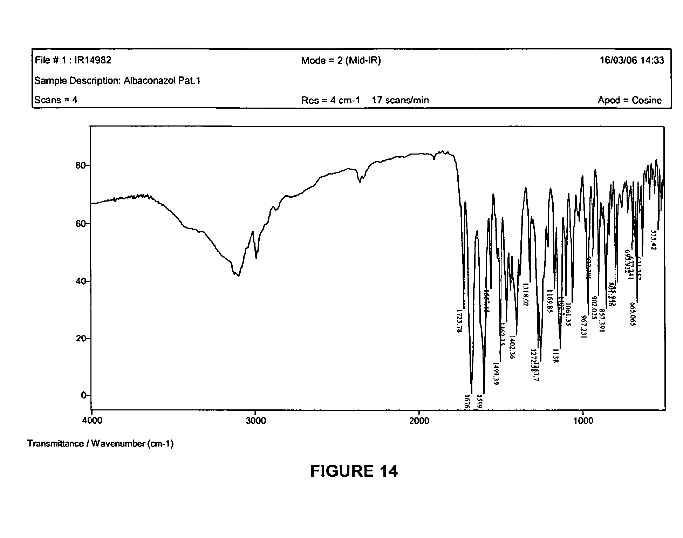

The complete infrared spectrum of the crystalline Form V is shown in Fig. 14 and is characterized as summarized below in Table 15.

**TABLE 15**

| **Infrared Spectrum for Form V** |
|---|
| **Frequency (cm⁻¹) (+/-0.1)** |
| 1723.8 |
| 1676.0 |
| 1599.0 |
| 1557.5 |
| 1499.4 |
| 1462.2 |
| 1402.4 |
| 1318.0 |
| 1272.4 |
| 1253.7 |
| 1169.9 |
| 1138.0 |
| 1102.7 |
| 1061.4 |
| 967.2 |
| 932.8 |
| 902.0 |
| 857.4 |
| 801.2 |
| 785.9 |
| 693.9 |
| 677.2 |
| 665.1 |
| 631.8 |
| 533.4 |

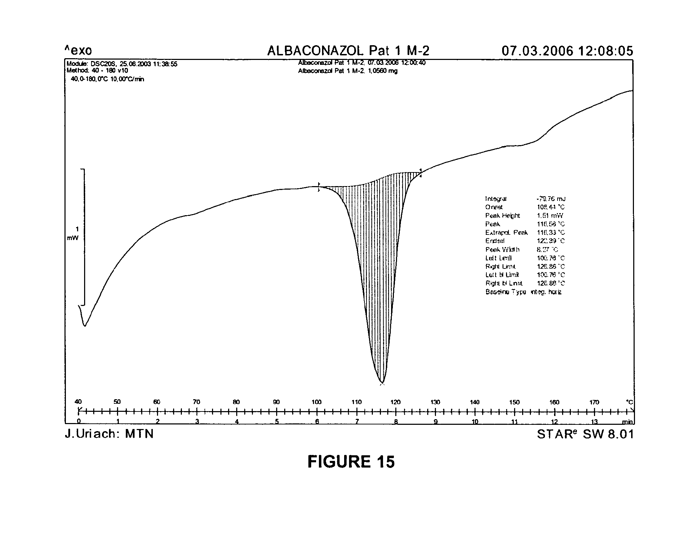

Form V was further observed to have a characteristic endothermic peak onset observed at about 108 +/- 5 degrees C using DSC analysis, as represented by Fig. 15.

### Pharmaceutical Compositions

In another preferred embodiment, the present subject matter relates to pharmaceutical compositions containing an anti-microbially or anti-fungally effective amount of any of the pure crystalline polymorphs I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one or a pharmaceutically acceptable salt or derivative thereof and a pharmaceutically acceptable carrier. In a particularly preferred embodiment, these pharmaceutical compositions comprise any one of the pure crystalline Forms I, II, III, IV, or V having a purity of at least 85% by weight, or not more than 15% by weight of other forms of the compound and any residual solvents. In a more preferred embodiment, these pharmaceutical compositions comprise any one of the pure crystalline Forms I, II, III, IV, or V having a purity of at least 90% by weight, with a purity of at least 95% by weight being most preferred.

The phrase "pharmaceutically acceptable carrier" as used in this regard refers to any inactive ingredient present in one of the herein described compositions in an amount effective to enhance the stability, effectiveness, or otherwise of said composition. Non-limiting examples of such pharmaceutically acceptable carriers include diluents, excipients, suspending agents, lubricating agents, adjuvants, vehicles, delivery systems, emulsifiers, disintegrants, absorbants, adsorbents, preservatives, surfactants, colorants, flavorants, emollients, buffers, pH modifiers, thickeners, water softening agents, humectants, fragrances, stabilizers, conditioning agents, chelating agents, sweeteners, propellants, anticaking agents, viscosity increasing agents, solubilizers, plasticizers, penetration enhancing agents, glidants, film forming agents, fillers, coating agents, binders, antioxidants, stiffening agents, wetting agents, or any mixture of these components.

The carriers useful herein may further include one or more compatible solid or liquid filler, diluents, or encapsulating materials which are suitable for human or animal administration.

Biocompatible carriers, as used herein, are components that do not cause any interactions which substantially reduce the efficacy of the pharmaceutical composition in an ordinary user environment. Possible pharmaceutical carriers must be of sufficiently low toxicity to make them suitable for administration to the subject of treatment.

Some non-limiting examples of substances which can serve as a carrier herein include sugar, starch, cellulose and its derivatives, powered tragacanth, malt, gelatin, talc, stearic acid, magnesium stearate, calcium sulfate, vegetable oils, polyols, alginic acid, pyrogen-free water, isotonic saline, phosphate buffer solutions, cocoa butter (suppository base), emulsifier as well as other non-toxic pharmaceutically compatible substances used in other pharmaceutical formulations. Wetting agents and lubricants such as sodium lauryl sulfate, as well as coloring agents, flavoring agents, excipients, tabletting agents, stabilizers, antioxidants, and preservatives may also be present.

Any non-toxic, inert, and effective carrier may be used to formulate the compositions contemplated herein. Suitable pharmaceutically acceptable carriers, excipients, and diluents in this regard are well known to those of skill in the art, such as those described in The Merck Index, Thirteenth Edition, Budavari et al., Eds., Merck & Co., Inc., Rahway, N.J. (2001); the CTFA (Cosmetic, Toiletry, and Fragrance Association) International Cosmetic Ingredient Dictionary and Handbook, Tenth Edition (2004); and the "Inactive Ingredient Guide", U.S. Food and Drug Administration (FDA) Center for Drug Evaluation and Research (CDER) Office of Management, http://www.accessdata.fda.gov/scripts/cder/iig/index.cfm, the contents of which are hereby incorporated by reference in their entirety. Examples of preferred pharmaceutically acceptable excipients, carriers and diluents useful in the present compositions include distilled water, physiological saline, Ringer's solution, dextrose solution, Hank's solution, and DMSO.

These additional inactive components, as well as effective formulations and administration procedures, are well known in the art and are described in standard textbooks, such as Goodman and Gillman's: The Pharmacological Bases of Therapeutics, 8th Ed., Gilman et al. Eds. Pergamon Press (1990) and Remington's Pharmaceutical Sciences, 18th Ed., Mack Publishing Co., Easton, Pa. (1990), both of which are incorporated by reference herein in their entirety.

The carrier may comprise, in total, from about 0.1% to about 99.99999% by weight of the pharmaceutical compositions presented herein, mainly from about 50% to about 99.9999%.

Preferred compositions herein can be in the form of oral or topical compositions. The oral compositions contemplated herein may take the form of tablets, capsules, soft-gels, hard gels, solutions, suspensions, powders, dispersible granules, cachets, combinations thereof, or any other oral pharmaceutical dosage form as would commonly be known in the art.

A solid carrier can be one or more substances which may also act as diluents, flavoring agents, solubilizers, lubricants, suspending agents, binders, or tablet disintegration agents; it can also be an encapsulating material. In powders, the carrier can be a finely divided solid which is in admixture with the active compound. In a tablet, the active compound can be mixed with a carrier having the necessary binding properties in suitable proportions and compacted in the size and shape desired. Non-limiting examples of suitable solid carriers include magnesium carbonate, magnesium stearate, talc, sugar, lactose, pectin, dextrin, starch, gelatin, tragacanth, methyl cellulose, sodium carboxymethylcellulose, hydroxypropyl methylcellulose, hydroxyethylcellulose, hydroxypropylcellulose, other cellulose derivatives, a low melting wax, cocoa butter, and the like.

Similarly, the topical compositions contemplated herein may take the form of a gel, cream, lotion, suspension, emulsion, aerosol, ointment, foam, mousse, shampoo, nail lacquer, nail product, vaginal product, combinations thereof, or any other topical pharmaceutical dosage form commonly known in the art. Other pharmaceutical and cosmetic treatment compositions known to those skilled in the art, including liquids and balms, are additionally contemplated as falling within the scope of the present subject matter. Further, the present subject matter contemplates applying any of these compositions with an applicator. Non-limiting examples of useful applicators in this regard include a pledget, a pad, and combinations thereof. Additionally, the present subject matter further contemplates that any of these topical compositions can be provided in a package of less than 5 g topical composition as a unit of use.

Emulsions, such as oil-in-water or water-in-oil systems, as well as a base (vehicle or carrier) for the topical formulation can be selected to provide effectiveness of the active ingredient and/or avoid allergic and irritating reactions (e.g., contact dermatitis) caused by ingredients of the base or by the active ingredients.

In severe cases, occlusive therapy may be useful herein. Covering the treated area with a nonporous occlusive dressing can increase the absorption and effectiveness of the compounds and compositions described herein. Usually, a polyethylene film (plastic household wrap) is applied overnight over cream or ointment, since a cream or ointment is usually less irritating than lotion in occlusive therapy. Plastic tapes may be impregnated with drug and are especially convenient for treating isolated or recalcitrant lesions; children and (less often) adults may experience pituitary and adrenal suppression after prolonged occlusive therapy over large areas.

Suitable gelling agents which may be useful in the present compositions include but are not limited to aqueous gelling agents, such as neutral, anionic, and cationic polymers, and mixtures thereof. Exemplary polymers which may be useful in the instant compositions include carboxy vinyl polymers, such as carboxypolymethylene. A preferred gelling agent is Carbopol® brand polymer such as is available from Noveon Inc., Cleveland, OH. Carbopol® polymers are high molecular weight, crosslinked, acrylic acid-based polymers. Carbopol® homopolymers are polymers of acrylic acid crosslinked with allyl sucrose or allylpentaerythritol. Carbopol® copolymers are polymers of acrylic acid, modified by long chain (C10-C30) alkyl acrylates, and crosslinked with allyl-pentaerythritol.

Other suitable gelling agents include cellulosic polymers, such as gum arabic, gum tragacanth, locust bean gum, guar gum, xanthan gum, cellulose gum, methylcellulose, hydroxyethylcellulose, hydroxypropylcellulose, and hydroxypropylmethylcellulose.

### Methods of Treatment

In another preferred embodiment, the present subject matter relates to a method of treating and/or preventing microbial or fungal infections or disorders in a mammal comprising administering to a mammal in need thereof an effective amount of any of the crystalline forms I, II, III, IV, or V of (1R,2R)-7-chloro-3-[2-(2,4-difluorophenyl)-2-hydroxy-1-methyl-3-(1H-1,2,4-triazol-1-yl)propyl]quinazolin-4(3H)-one or a pharmaceutically acceptable salt or derivative thereof. In a particularly preferred embodiment, these methods comprise the use of any one of the pure crystalline Forms I, II, III, IV, or V having a purity of at least 85% by weight, or having not more than 15% by weight of other forms of the compound and any residual solvents, with a purity of at least 90% by weight being most preferred. In an especially preferred embodiment, the present crystalline Forms I, II, III, IV, or V can be used in a method of treating and/or preventing Chagas Disease in a mammal.

In another preferred embodiment, the present methods of treatment result in an improvement of the patient's condition, reduction of symptoms, an improvement in the patient's appearance, or combinations thereof.

In preferred embodiments, the administration of the present compositions is capable of reducing the number of microbes, preferably pathogenic microbes, in or on the mammal to which it is administered. The microbes that can be acted on by the present compositions are selected from the group consisting of fungi, molds, and combinations thereof. The present compositions are further capable of treating infections of the parasite *Trypanosoma cruzi.*

Preferred, non-limiting examples of such fungi are those selected from the group consisting of *P. ovale, P. oviculare, M. furfur, C. neoformans, S. prolificans, S. shenkii, Epidermophyton floccosum, Microsporum canis, Candida* sp., *Trichophyton* sp., and combinations thereof.

Preferred, non-limiting examples of such *Candida* sp. are those selected from the group consisting of *C. albicans, C. krusei, C, glabrata, C. guillermondii, C. parapsilosis, C. tropicalis,* and combinations thereof.

Preferred, non-limiting examples of such *Trichophyton* sp. are those selected from the group consisting of *T. rubrum, T. mentagrophytes, T. tonsurans, T. violaceum,* and combinations thereof.

Preferred, non-limiting examples of such molds are *Aspergillus* sp.

Preferred, non-limiting examples of such *Aspergillus* sp. are those selected from the group consisting of *A. flavus, A. fumigatus, A. niger,* and combinations thereof.

Several specific skin disorders may also be treated according to the present methods. Exemplary among these skin disorders are seborrheic dermatitis, *Pityrosporum* infections, tinea versicolor, tinea pedis, tinea cruris, tinea corporis, cutaneous candidiasis, onychomycosis, and combinations thereof. Other skin disorders known to those of ordinary skill in the art as effectively treatable by an antimicrobial or antifungal composition are further contemplated as within the scope of the present subject matter.

Further, the present methods provide antimycotic activity against *Pityrosporum* strains, such as *Pityrosporum ovale* and *Pityriasis versicolor.*

In this regard, the present compositions are particularly effective in treating the specific dermatophytes *Tinea* and/or *Candida* fungi.

### Combination Therapy

In another preferred embodiment, the present preferred compositions may be used in combination with an additional pharmaceutical dosage form to enhance their effectiveness in treating a microbial or fungal infection or disorder. In this regard, the present preferred compositions may be administered as part of a regimen additionally including any other pharmaceutical and/or pharmaceutical dosage form known in the art as effective for the treatment of a microbial and/or fungal infection. Similarly, a pharmaceutically active ingredient other than those specified herein can be added to the present preferred compositions to enhance their effectiveness in treating a microbial and/or fungal infection or disorder. Accordingly, this additional pharmaceutically active ingredient or additional pharmaceutical dosage form can be administered to a patient either directly or indirectly, and concomitantly or sequentially, with the preferred compositions described herein.

In this regard, antimicrobial agents other than the (1R,2R)-7-chloro-3-[2-(2,4-difluorophenyl)-2-hydroxy-1-methyl-3-(1H-1,2,4-triazol-1-yl)propyl]quinazolin-4(3H)-one discussed above are additionally contemplated as useful for combination therapy discussed herein. Included among these other antimicrobial agents are those selected from the group consisting of imidazoles, allylamines, triazoles, glucan synthase inhibitors, chitin synthase inhibitors, polyenes, griseofulvin, morpholine derivatives, triazines, pyrimidines, any other antimicrobial azole, pharmaceutically acceptable salts or derivatives thereof, and mixtures thereof. Other antimicrobial agents known in the art as effective upon administration to a patient are further contemplated as effective within the present subject matter.

In a preferred embodiment, these other antimicrobial agents are those selected from the group consisting of amorolfine, amphotericin B, bacitracin, benzalkonium chloride, benzethonium chloride, bifonazole, butenafine, butoconazole, chloroxine, cilofungin, chlordantoin, chlortetracycline, ciclopirox, clindamycin, clioqinol, clotrimazole, econazole, elubiol, faeriefungin, fezatione, fluconazole, flucytosine, fungimycin, gentamicin, griseofulvin, haloprogin, hexylresorcinol, itraconazole, ketoconazole, methylbenzethonium chloride, miconazole, mupirocin, naftifine, nikkomycin Z, nystatin, I-ofloxacin, oxiconazole, oxytetracycline, phenol, polymyxin B, pyrido[3,4-e]-1,2,4-triazine, pyrrolnitrin, quaternary ammonium compounds, salicylic acid, saperconazole, sulconazole, tea tree oil, terbinafine, terconazole, tetracyclines, thiabendazole, ticlatone, tioconazole, tolnaftate, triacetin, triclocarbon, triclosan, undecylenic acid, voriconazole, zinc and sodium pyrithione, a pharmaceutically acceptable salt or derivative thereof, and a mixture thereof. Combinations of any of the foregoing anti-microbial agents or their pharmaceutically acceptable salts or derivatives are contemplated herein.

In one embodiment in this regard, the present preferred compositions and the additional pharmaceutical dosage form can be administered to a patient at the same time. In an alternative embodiment, one of the present preferred compositions and the additional pharmaceutical dosage form can be administered in the morning and the other can be administered in the evening.

In another preferred embodiment, the presently described compounds can be administered to a patient in need thereof in multiple pharmaceutical dosage forms. This combination therapy may maximize the effectiveness of the present composition in treating a microbial or fungal infection or disorder. In one preferred embodiment in this regard, both an oral and a topical composition, each containing form III of (1R,2R)-7-chloro-3-[2-(2,4-difluorophenyl)-2-hydroxy-1-methyl-3-(1H-1,2,4-triazol-1-yl)propyl]quinazolin-4(3H)-one, can be administered, either concomitantly or sequentially, to a patient suffering from a microbial and/or fungal infection or disorder. In the alternative, the oral and topical compositions can contain different amorphous and/or crystalline forms of the (1R,2R)-7-chloro-3-[2-(2,4-difluorophenyl)-2-hydroxy-1-methyl-3-(1H-1,2,4-tiazol-1-yl)propyl]quinazolin-4(3H)-one compound.

### Methods of Production

Each of the crystalline forms I, II, III, IV, and V of (1R,2R)-7-chloro-3-[2-(2,4-difluorophenyl)-2-hydroxy-1-methyl-3-(1H-1,2,4-triazol-1-yl)propyl]quinazolin-4(3H)-one can be prepared by a separate process to arrive at a separate crystalline form.

For example, the present subject matter relates to a process for preparing a crystalline Form I or II of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
crystallizing a crystalline Form I or II of (1R,2R)-7-chloro-3-[2-(2,4-difluorophenyl)-2-hydroxy-1-methyl-3-(1H-1,2,4-triazol-1-yl)propyl]quinazolin-4(3H)-one from an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one using supercritical CO₂ crystallization conditions.

Similarly, the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising recrystallizing (1R,2R)-7-chloro-3-[2-(2,4-difluorophenyl)-2-hydroxy-1-methyl-3-(1H-1,2,4-triazol-1-yl)propyl]quinazolin-4(3H)-one from a solution or suspension of (1R,2R)-7-chloro-3-[2-(2,4-difluorophenyl)-2-hydroxy-1-methyl-3-(1H-1,2,4-triazol-1-yl)propyl]quinazolin-4(3H)-one in an organic solvent, particularly an organic solvent selected from the group consisting of ethanol, ethyl acetate, dichloromethane, and a combination of ethanol and ethyl acetate. In a most preferred embodiment, the organic solvent is ethanol. In a further preferred embodiment, the (1R,2R)-7-chloro-3-[2-(2,4-difluorophenyl)-2-hydroxy-1-methyl-3-(1H-1,2,4-triazol-1-yl)propyl]quinazolin-4(3H)-one forms a solution in the organic solvent.

Further, the solution or suspension is preferably formed at a temperature of at least 40° C. This solution or suspension can then optionally be cooled to a temperature of about 10° C to about 20° C. Accordingly, the present subject matter relates to a crystalline form of (1R,2R)-7-chloro-3-[2-(2,4-difluorophenyl)-2-hydroxy-1-methyl-3-(1H-1,2,4-triazol-1-yl)propyl]quinazolin-4(3H)-one comprising the reaction product of (1R,2R)-7-chloro-3-[2-(2,4-difluorophenyl)-2-hydroxy-1-methyl-3-(1H-1,2,4-triazol-1-yl)propyl]quinazolin-4(3H)-one and an organic solvent, particularly an organic solvent selected from the group consisting of ethanol, ethyl acetate, dichloromethane, and a combination of ethanol and ethyl acetate, with ethanol being most preferred.

In a further preferred embodiment in this regard, the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
adding (1R,2R)-7-chloro-3-[2-(2,4-difluorophenyl)-2-hydroxy-1-methyl-3-(1H-1,2,4-triazol-1-yl)propyl]quinazolin-4(3H)-one to a solvent selected from the group consisting of ethanol, ethyl acetate, dichloromethane, and a combination of ethanol and ethyl acetate to form a solution or suspension; and
crystallizing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one from said solution or suspension.

In a particularly preferred embodiment in this regard, the (1R,2R)-7-chloro-3-[2-(2,4-difluorophenyl)-2-hydroxy-1-methyl-3-(1H-1,2,4-triazol-1-yl)propyl]quinazolin-4(3H)-one added to the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. In a further particularly preferred embodiment, the solvent is ethanol and is used to form a solution of (1R,2R)-7-chloro-3-[2-(2,4-difluorophenyl)-2-hydroxy-1-methyl-3-(1H-1,2,4-triazol-1-yl)propyl]quinazolin-4(3H)-one.

In still another preferred embodiment, the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in a solvent selected from the group consisting of ethanol, ethyl acetate, dichloromethane, and a combination of ethanol and ethyl acetate to form a solution;
crystallizing crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one; and
drying said crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

In a preferred embodiment, the (1R,2R)-7-chloro-3-[2-(2,4-difluorophenyl)-2-hydroxy-1-methyl-3-(1H-1,2,4-triazol-1-yl)propyl]quinazolin-4(3H)-one added to the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. Further in this regard, the crystalline Form III is preferably first filtered and then heated to about 65° C to about 75° C in a vacuum to reduce the solvent content. In a preferred embodiment, the crystalline Form III is heated to about 70° C. And again, the most preferred solvent used in this regard is ethanol.

In an alternative embodiment, this process further comprises the step of refluxing the solution before crystallizing form III. In a further alternative embodiment, this process comprises the additional step of filtering the solution after it is refluxed.

In a further preferred embodiment, the present subject matter relates to a process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
adding (1R,2R)-7-chloro-3-[2-(2,4-difluorophenyl)-2-hydroxy-1-methyl-3-(1H-1,2,4-triazol-1-yl)propyl]quinazolin-4(3H)-one to an ethanol solvent to form a solution or suspension;
crystallizing crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one; and
drying said crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

Likewise, the present subject matter relates to a process for preparing a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
storing an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one for 3 months at about 40°C and 75% HR;
transforming some of said amorphous (1R,2R)-7-chloro-3-[2-(2,4-difluorophenyl)-2-hydroxy-1-methyl-3-(1H-1,2,4-triazol-1-yl)propyl]quinazolin-4(3H)-one to a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one during storage; and
obtaining said crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

In this regard, this process results in a mixture of both the amorphous form and Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

In another preferred embodiment in this regard, the present subject matter relates to an alternative process for preparing a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in ethanol to form a solution;
adding the solution to water to form a suspension;
stirring the suspension for more than 30 minutes;
obtaining crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one; and
separating said crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

In a particularly preferred embodiment in this regard, the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. However, any form of (1R,2R)-7-chloro-3-[2-(2,4-difluorophenyl)-2-hydroxy-1-methyl-3-(1H-1,2,4-triazol-1-yl)propyl]quinazolin-4(3H)-one my be suitable for this purpose.

In this regard, the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one is preferably dissolved in hot ethanol, i.e. ethanol at a temperature of at least 40° C. This solution can then preferably be added to the water at about 5° C to about 10° C. In an alternative embodiment, the solution can be added to the water at room temperature. Further, the suspension is preferably stirred for at least 6 hours. In a particularly preferred embodiment in this regard, the suspension is stirred for at least 18 hours.

In another preferred embodiment, the present subject matter relates to a process for preparing a crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in ethyl acetate to form a solution;
adding hexane to the solution;
optionally adding diethyl ether to the solution;
crystallizing Form V of (1R,2R)-7-chloro-3-[2-(2,4-difluorophenyl)-2-hydroxy-1-methyl-3-(1H-1,2,4-triazol-1-yl)propyl]quinazolin-4(3H)-one; and
separating said crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

In a particularly preferred embodiment in this regard, the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one. However, any form of (1R,2R)-7-chloro-3-[2-(2,4-difluorophenyl)-2-hydroxy-1-methyl-3-(1H-1,2,4-triazol-1-yl)propyl]quinazolin-4(3H)-one my be suitable for this purpose.

In this regard, the amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one is preferably dissolved in hot ethyl acetate, i.e. ethyl acetate at a temperature of at least 40° C. Further, the solution can preferably be cooled after addition of the hexane.

### Dosage

Appropriate dosage levels for any of the crystalline forms of any of the active ingredients and/or their specific crystal forms are well known to those of ordinary skill in the art and are selected to maximize the treatment of the previously described microbial and/or fungal conditions. Dosage levels on the order of about 0.001 mg to about 5,000 mg per kilogram body weight of the active ingredient components are known to be useful in the treatment of the diseases, disorders, and conditions contemplated herein. Typically, this effective amount of the active agent will generally comprise from about 0.001 mg to about 100 mg per kilogram of patient body weight per day. Moreover, it will be understood that this dosage of ingredients can be administered in a single or multiple dosage units to provide the desired therapeutic effect.

If desired, other therapeutic agents can be employed in conjunction with those provided in the above-described compositions. The amount of pharmaceutically active ingredients that may be combined with the carrier materials to produce a single dosage form will vary depending upon the host treated, the nature of the disease, disorder, or condition, and the nature of the active ingredients.

The preferred pharmaceutical compositions may be given in a single or multiple doses daily. In a preferred embodiment, the pharmaceutical compositions are given from one to three times daily. Starting with a low dose twice daily and slowly working up to higher doses if needed is a preferred strategy. The amount of pharmaceutically active ingredients that may be combined with the carrier materials to produce a single dosage form will vary depending upon the host treated, the nature of the disease, disorder, or condition, and the nature of the active ingredients.

It is understood, however, that a specific dose level for any particular patient will vary depending upon a variety of factors, including the activity of the specific pharmaceutically active agent; the age, body weight, general health, sex and diet of the patient; the time of administration; the rate of excretion; possible drug combinations; the severity of the particular condition being treated; and the form of administration. One of ordinary skill in the art would appreciate the variability of such factors and would be able to establish specific dose levels using no more than routine experimentation.

The optimal pharmaceutical formulations will be determined by one skilled in the art depending upon considerations such as the particular pharmaceutically active agent combination and the desired dosage. See, for example, "Remington's Pharmaceutical Sciences", 18th ed. (1990, Mack Publishing Co., Easton, PA 18042), pp. 1435-1712, the disclosure of which is hereby incorporated by reference. Such formulations may influence the physical state, stability, rate of *in vivo* release, and rate of *in vivo* clearance of the essential lipids.

### EXAMPLES

The presently presented compounds can be prepared in accordance with the following Examples using commercially available starting materials. The (1R,2R)-7-chloro-3-[2-(2,4-difluorophenyl)-2-hydroxy-1-methyl-3-(1H-1,2,4-triazol-1-yl)propyl]quinazolin-4(3H)-one used in the foregoing examples may be obtained by any of the currently available processes that are well known in the art, such as, for example, the processes described in Bartroli et al., J. Med. Chem., Vol. 41, No. 11, pp. 1869-1882 (1998), the contents of which are hereby incorporated by reference in their entirety.

In solution, no crystalline form exists, and thus the physicochemical solution characteristics, i.e. ¹H NMR spectra, ultraviolet spectra, and specific rotations of the crystalline and amorphous forms of (1R,2R)-7-chloro-3-[2-(2,4-difluorophenyl)-2-hydroxy-1-methyl-3-(1H-1,2,4-triazol-1-yl)propyl]quinazolin-4(3H)-one are the same.

### EXAMPLE 1

Preparation of crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one

1.5 g of amorphous (1R,2R)-7-chloro-3-[2-(2,4-difluorophenyl)-2-hydroxy-1-methyl-3-(1H-1,2,4-triazol-1-yl)propyl]quinazolin-4(3H)-one was dispersed in a column filled with glass beads which act as a disaggregating agent. The column was inserted in an extractor and a flux of supercritical CO₂ (SCCO2) was passed (p=250 bar, T=45°C). A filter was installed at the extractor exit to collect the material once dissolved by the SCCO2. Only 0.15 g of material remained in the column. This material was crystalline, and was shown to have crystalline Form I.

### EXAMPLE 2

Preparation of crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one

0.5 g of amorphous (1R,2R)-7-chloro-3-[2-(2,4-difluorophenyl)-2-hydroxy-1-methyl-3-(1H-1,2,4-triazol-1-yl)propyl]quinazolin-4(3H)-one was dissolved in 200 ml of ethanol. The solution was put in a reactor which was pressurized with CO₂ up to p=100 bar and T=40°C (supercritical conditions). The reactor was depressurized, and any significant amount of product was collected in the outer filter. Once the ethanol was evacuated, 0.52 g of resulting material was collected. The X-ray diffractogram of this sample showed a crystalline pattern. This material was shown to have crystalline Form II.

### EXAMPLE 3

The procedure of Example 1 was repeated but substituting the glass beads with silica sand. The supercritical conditions were p=250 bar and T=52°C. In this case, sample could be collected in the outer filer, which was of crystalline Form II, as well as inside the reactor, which was of crystalline Form I.

### EXAMPLE 4

Preparation of crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one

Amorphous (1R,2R)-7-chloro-3-[2-(2,4-difluorophenyl)-2-hydroxy-1-methyl-3-(1H-1,2,4-triazol-1-yl)propyl]quinazolin-4(3H)-one was dissolved in ethanol and recrystallized. The original ¹H NMR spectrum showed about ½ mol of ethanol. The sample was kept in a capped vial at room temperature for 7 years. After 7 years, the samples NMR spectrum showed a total loss of ethanol. The sample was then shown to have crystalline Form III.

### EXAMPLE 5

The procedure of Example 4 was repeated but substituting the ethanol with ethanol and ethyl acetate, ethyl acetate, or dichloromethane. In each case, the recrystallized sample was shown to have crystalline Form III.

### EXAMPLE 6

Preparation of crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one

6.785 kg of (1R,2R)-7-chloro-3-[2-(2,4-difluorophenyl)-2-hydroxy-1-methyl-3-(1H-1,2,4-triazol-1-yl)propyl]quinazolin-4(3H)-one was dissolved in 17.3 L of ethanol. The solution was cooled at 10-20°C for about 2 hours. The obtained product was centrifuged and dried in vacuum at 70°C to obtain 5.796 kg of crystalline (1R,2R)-7-chloro-3-[2-(2,4-difluorophenyl)-2-hydroxy-1-methyl-3-(1H-1,2,4-triazol-1-yi)propyl]quinazolin-4(3H)-one. The product was then shown to have crystalline Form III.

### EXAMPLE 7

Preparation of crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one

(1R,2R)-7-chloro-3-[2-(2,4-difluorophenyl)-2-hydroxy-1-methyl-3-(1H-1,2,4-triazol-1-yl)propyl]quinazolin-4(3H)-one was dissolved in an excess of hot ethanol. Charcoal was added and the solution was filtered. The filtered solution was then concentrated in vacuum to a final volume of 2.5-3 L/kg of (1R,2R)-7-chloro-3-[2-(2,4-difluorophenyl)-2-hydroxy-1-methyl-3-(1H-1,2,4-triazol-1-yl)propyl]quinazolin-4(3H)-one. The concentrated solution was then cooled to 5-10°C and (1R,2R)-7-chloro-3-[2-(2,4-difluorophenyl)-2-hydroxy-1-methyl-3-(1H-1,2,4-triazol-1-yl)propyl]quinazolin-4(3H)-one crystallized. The obtained product was filtered and dried in vacuum at 70°C to obtain crystalline (1R,2R)-7-chloro-3-[2-(2,4-difluorophenyl)-2-hydroxy-1-methyl-3-(1H-1,2,4-triazol-1-yl)propyl]quinazolin-4(3H)-one. The product was then shown to have crystalline Form III.

### EXAMPLE 8

Preparation of crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one

8.10 g of (1R,2R)-7-chloro-3-[2-(2,4-difluorophenyl)-2-hydroxy-1-methyl-3-(1H-1,2,4-triazol-1-yl)propyl]quinazolin-4(3H)-one were dissolved in 65 mL of hot ethanol. This solution was added to 400 mL of water at about 5 to about 10° C. The suspension obtained was stirred for 18 hours. 7.07 g of (1R,2R)-7-chloro-3-[2-(2,4-difluorophenyl)-2-hydroxy-1-methyl-3-(1H-1,2,4-triazol-1-yl)propyl]quinazolin-4(3H)-one were obtained. The product was then shown to have crystalline Form IV.

### EXAMPLE 9

Preparation of crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one

18.15 g of (1R,2R)-7-chloro-3-[2-(2,4-difluorophenyl)-2-hydroxy-1-methyl-3-(1H-1,2,4-triazol-1-yl)propyl]quinazolin-4(3H)-one were dissolved in 130 mL of hot ethanol. This solution was added to 800 mL of water at room temperature. The suspension obtained was stirred for 25 hours at room temperature. The product was filtered and dried in vacuum at 50°C for 18 hours. 16.98 g of (1R,2R)-7-chloro-3-[2-(2,4-difluorophenyl)-2-hydroxy-1-methyl-3-(1H-1,2,4-triazol-1-yl)propyl]quinazolin-4(3H)-one were obtained. The product was then shown to have crystalline Form IV.

### EXAMPLE 10

Preparation of crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one

10.0 g of (1R,2R)-7-chloro-3-[2-(2,4-difluorophenyl)-2-hydroxy-1-methyl-3-(1H-1,2,4-triazol-1-yl)propyl]quinazolin-4(3H)-one were dissolved in 30 mL of hot ethyl acetate. 10 mL of Hexane were then added and the solution was slightly cooled. Crystallization was immediately observed. 20 mL of diethyl ether were then added, and the suspension was stirred on an ice bath for one hour. The obtained product was filtered and dried in vacuum at about 40° C for 8 hours. The product was then dried at 60° C in vacuum for 24 hours. The product was then shown to have crystalline Form V.

### EXAMPLE 11

A patient is suffering from onychomycosis. A preferred composition herein is administered to the patient. It would be expected that the patient would improve his/her condition or recover.

### EXAMPLE 12

A patient is suffering from Chagas Disease. A preferred composition herein is administered to the patient. It would be expected that the patient would improve his/her condition or recover.

The present subject matter being thus described, it will be apparent that the same may be modified or varied in many ways. Such modifications and variations are not to be regarded as a departure from the spirit and scope of the present subject matter, and all such modifications and variations are intended to be included within the scope of the following claims.

## Claims

1. A crystalline form of (1R,2R)-7-chloro-3-[2-(2,4-difluorophenyl)-2-hydroxy-1-methyl-3-(1H-1,2,4-triazol-1-yl)propyl]quinazolin-4(3H)-one.

2. The crystalline form of claim 1, which comprises consistent chemical, physical, mechanical, electrical, thermodynamic, and biological characteristics selected from the group consisting of storage stability, solubility, melting point, chemical stability, physical stability, and combinations thereof.

3. A crystalline form of (1R,2R)-7-chloro-3-[2-(2,4-difluorophenyl)-2-hydroxy-1-methyl-3-(1H-1,2,4-triazol-1-yl)propyl]quinazolin-4(3H)-one comprising the reaction product of (1R,2R)-7-chloro-3-[2-(2,4-difluorophenyl)-2-hydroxy-1-methyl-3-(1H-1,2,4-triazol-1-yl)propyl]quinazolin-4(3H)-one and an organic solvent.

4. The crystalline form of claim 3, wherein said organic solvent is selected from the group consisting of ethanol, ethyl acetate, dichloromethane, and a combination of ethanol and ethyl acetate.

5. The crystalline form of claim 4, wherein said organic solvent is ethanol.

6. A substantially pure crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one.

7. The substantially pure crystalline Form I of claim 6, wherein the crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one has characteristic X-ray powder diffraction (XRPD) 2-theta positions at about 4.11, 8.22, 9.39, 11.29, 12.41, 13.62, 16.74, 17.57, 18.79, 23.82, and 25.2 +/- 0.2.

8. The substantially pure crystalline Form I of claim 6 wherein the crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one is **characterized by** at least one of the following properties:
an X-ray powder diffraction pattern substantially similar to that presented in Fig. 1 or
a differential scanning calorimetry thermogram substantially similar to that presented in Fig. 3.

9. A pharmaceutical composition comprising an anti-microbially or anti-fungally effective amount of the substantially pure crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 6 or a pharmaceutically acceptable salt or derivative thereof and a pharmaceutically acceptable carrier.

10. A method of treating and/or preventing microbial or fungal infections in a mammal comprising administering to a mammal in need thereof an effective amount of the substantially pure crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 6 or a pharmaceutically acceptable salt or derivative thereof.

11. A method of treating and/or preventing Chagas Disease in a mammal comprising administering to a mammal in need thereof an effective amount of the substantially pure crystalline Form I of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 6 or a pharmaceutically acceptable salt or derivative thereof.

12. A substantially pure crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one.

13. The substantially pure crystalline Form II of claim 12, wherein the crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one has characteristic X-ray powder diffraction (XRPD) 2-theta positions at about 6.35, 7.98, 8.37, 11.4, 11.7, 12.6, 13.15, 14.42, 14.98, 16.77, 17.56, 18.86, 21.08, 23.84, 25.32, 26.83, and 27.35 +/- 0.2.

14. The substantially pure crystalline Form II of claim 12, wherein the crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one is **characterized by** at least one of the following properties:
an X-ray powder diffraction pattern substantially similar to that presented in Fig. 4; or
a differential scanning calorimetry thermogram substantially similar to that presented in Fig. 6.

15. A process for preparing a crystalline Form I or II of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
crystallizing a crystalline Form I or II of (1R,2R)-7-chloro-3-[2-(2,4-difluorophenyl)-2-hydroxy-1-methyl-3-(1H-1,2,4-triazol-1-yl)propyl]quinazolin-4(3H)-one from an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one using supercritical CO₂ crystallization conditions.

16. A pharmaceutical composition comprising an anti-microbially or anti-fungally effective amount of the substantially pure crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 12 or a pharmaceutically acceptable salt or derivative thereof and a pharmaceutically acceptable carrier.

17. A method of treating and/or preventing microbial or fungal infections in a mammal comprising administering to a mammal in need thereof an effective amount of the substantially pure crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 12 or a pharmaceutically acceptable salt or derivative thereof.

18. A method of treating and/or preventing Chagas Disease in a mammal comprising administering to a mammal in need thereof an effective amount of the substantially pure crystalline Form II of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 12 or a pharmaceutically acceptable salt or derivative thereof.

19. A substantially pure crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

20. The substantially pure crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 19, which has less than about 10% by weight of Forms I, II, IV, or V or an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one as determined on a % weight basis.

21. The substantially pure crystalline Form III of claim 19, wherein the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one has at least 90% purity as defined by X-ray powder diffraction.

22. The substantially pure crystalline Form III of claim 19, which has less than about 10% by weight of any residual solvent.

23. The substantially pure crystalline Form III of claim 22, which has less than about 5% by weight of any residual solvent.

24. The substantially pure crystalline Form III of claim 19, wherein the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one has characteristic X-ray powder diffraction (XRPD) 2-theta positions at about 4.08, 5.73, 6.22, 7.77, 8.15, 8.80, 11.25, 11.47, 12.44, 13.09, 15.57, 17.63, 18.66, 20.85, 26.65, and 27.12 +/- 0.2.

25. The substantially pure crystalline Form III of claim 19, wherein the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one is **characterized by** at least one of the following properties:
an X-ray powder diffraction pattern substantially similar to that presented in Fig. 7; or
a differential scanning calorimetry thermogram substantially similar to that presented in Fig. 9.

26. The substantially pure crystalline Form III of claim 19, wherein the crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one is storage stable for at least 6 months at about 25°C and 60% HR.

27. A process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising recrystallizing (1R,2R)-7-chloro-3-[2-(2,4-difluorophenyl)-2-hydroxy-1-methyl-3-(1H-1,2,4-triazol-1-yl)propyl]quinazolin-4(3H)-one from a solution or suspension of (1R,2R)-7-chloro-3-[2-(2,4-difluorophenyl)-2-hydroxy-1-methyl-3-(1H-1,2,4-triazol-1-yl)propyl]quinazolin-4(3H)-one in an organic solvent.

28. The process of claim 27, wherein said organic solvent is selected from the group consisting of ethanol, ethyl acetate, dichloromethane, and a combination of ethanol and ethyl acetate.

29. The process of claim 28, wherein said organic solvent is ethanol.

30. A process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
adding (1R,2R)-7-chloro-3-[2-(2,4-difluorophenyl)-2-hydroxy-1-methyl-3-(1H-1,2,4-triazol-1-yl)propyl]quinazolin-4(3H)-one to a solvent selected from the group consisting of ethanol, ethyl acetate, dichloromethane, and a combination of ethanol and ethyl acetate to form a solution or suspension; and
crystallizing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one from said solution or suspension.

31. A process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in a solvent selected from the group consisting of ethanol, ethyl acetate, dichloromethane, and a combination of ethanol and ethyl acetate thereof to form a solution;
crystallizing crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one; and
drying said crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

32. The process of claim 31, further comprising the step of refluxing the solution.

33. The process of claim 32, further comprising the step of filtering the solution.

34. The process of claim 31, wherein the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one.

35. A process for preparing a crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
adding (1R,2R)-7-chloro-3-[2-(2,4-difluorophenyl)-2-hydroxy-1-methyl-3-(1H-1,2,4-triazol-1-yl)propyl]quinazolin-4(3H)-one to an ethanol solvent to form a solution or suspension;
crystallizing crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one; and
drying said crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

36. A pharmaceutical composition comprising an anti-microbially or anti-fungally effective amount of the substantially pure crystalline Form III of (1R,2R)-7-chloro-3-[2-(2,4-difluorophenyl)-2-hydroxy-1-methyl-3-(1H-1,2,4-triazo)-1-yl]propyl]quinazolin-4(3H)-one of claim 19 or a pharmaceutically acceptable salt or derivative thereof and a pharmaceutically acceptable carrier.

37. The pharmaceutical composition of claim 36, wherein said (1R,2R)-7-chloro-3-[2-(2,4-difluorophenyl)-2-hydroxy-1-methyl-3-(1H-1,2,4-triazol-1-yl)propyl]quinazolin-4(3H)-one comprises at least 95% by weight of the substantially pure crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one.

38. A method of treating and/or preventing microbial or fungal infections in a mammal comprising administering to a mammal in need thereof an effective amount of the substantially pure crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 19 or a pharmaceutically acceptable salt or derivative thereof.

39. A method of treating and/or preventing Chagas Disease in a mammal comprising administering to a mammal in need thereof an effective amount of the substantially pure crystalline Form III of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 19 or a pharmaceutically acceptable salt or derivative thereof.

40. A substantially pure crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

41. The substantially pure crystalline Form IV of claim 40, wherein the crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one has characteristic X-ray powder diffraction (XRPD) 2-theta positions at about 4.15, 7.5, 8.33, 9.61, 11.16, 12.49, 13.29, 13.64, 14.41, 16.90, 18.74, 24.78, and 25.11 +/- 0.2.

42. The substantially pure crystalline Form IV of claim 40, wherein the crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one is **characterized by** at least one of the following properties:
an X-ray powder diffraction pattern substantially similar to that presented in Fig. 10; or
a differential scanning calorimetry thermogram substantially similar to that presented in Fig. 12.

43. A process for preparing a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
storing an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one for 3 months at 40°C and 75% HR;
transforming some of said amorphous (1R,2R)-7-chloro-3-[2-(2,4-difluorophenyl)-2-hydroxy-1-methyl-3-(1H-1,2,4-triazol-1-yl)propyl]quinazolin-4(3H)-one to a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one during storage; and
obtaining said polymorph Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

44. A process for preparing a crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in ethanol to form a solution;
adding the solution to water to form a suspension;
stirring the suspension for more than 30 minutes;
obtaining crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one; and
separating said crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one.

45. The process of claim 44, wherein the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in ethanol is an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one.

46. A pharmaceutical composition comprising an anti-microbially or anti-fungally effective amount of the substantially pure crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 40 or a pharmaceutically acceptable salt or derivative thereof and a pharmaceutically acceptable carrier.

47. A method of treating and/or preventing microbial or fungal infections in a mammal comprising administering to a mammal in need thereof an effective amount of the substantially pure crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 40 or a pharmaceutically acceptable salt or derivative thereof.

48. A method of treating and/or preventing Chagas Disease in a mammal comprising administering to a mammal in need thereof an effective amount of the substantially pure crystalline Form IV of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 40 or a pharmaceutically acceptable salt or derivative thereof.

49. A substantially pure crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

50. The substantially pure crystalline of claim 49, wherein the crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one has characteristic X-ray powder diffraction (XRPD) 2-theta positions at about 4.17, 5.83, 6.28, 7.96, 8.35, 11.35, 11.66, 12.57, 14.34, 14.89, 15.95, 16.74, 17.49, 18.9, 21.1, 23.86, 25.3, 27.04, 27.44, and 30.13 +/- 0.2.

51. The substantially pure crystalline Form IV of claim 49, wherein the crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one is **characterized by** at least one of the following properties:
an X-ray powder diffraction pattern substantially similar to that presented in Fig. 13; or
a differential scanning calorimetry thermogram substantially similar to that presented in Fig. 15.

52. A process for preparing a crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one comprising:
dissolving (1R,2R)-7-chloro-3-[2-(2,4-difluorophenyl)-2-hydroxy-1-methyl-3-(1H-1,2,4-triazol-1-yl)propyl]quinazolin-4(3H)-one in ethyl acetate to form a solution;
adding hexane to the solution;
optionally adding diethyl ether to the solution;
crystallizing crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one; and
separating said crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one.

53. The process of claim 52, wherein the (1R,2R)-7-chloro-3-[2-(2,4-difluorophenyl)-2-hydroxy-1-methyl-3-(1H-1,2,4-triazol-1-yl)propyl]quinazolin-4(3H)-one dissolved in the solvent is an amorphous form of (1R,2R)-7-chloro-3-[2-(2,4-difluorophenyl)-2-hydroxy-1-methyl-3-(1H-1,2,4-triazol-1-yl)propyl]quinazolin-4(3H)-one.

54. A pharmaceutical composition comprising an anti-microbially or anti-fungally effective amount of the substantially pure crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 49 or a pharmaceutically acceptable salt or derivative thereof and a pharmaceutically acceptable carrier.

55. A method of treating and/or preventing microbial or fungal infections in a mammal comprising administering to a mammal in need thereof an effective amount of the substantially pure crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 49 or a pharmaceutically acceptable salt or derivative thereof.

56. A method of treating and/or preventing Chagas Disease in a mammal comprising administering to a mammal in need thereof an effective amount of the substantially pure crystalline Form V of (1R,2R)-7-chloro-3-[2-(2,4-difluorophenyl)-2-hydroxy-1-methyl-3-(1H-1,2,4-triazol-1-yl)propyl]quinazolin-4(3H)-one of claim 49 or a pharmaceutically acceptable salt or derivative thereof.
